(19)

Europäisches
Patentamt

European
Patent Office

Office européen
des brevets

(11) **EP 3 959 512 B1**

(12) **EUROPEAN PATENT SPECIFICATION**

(45) Date of publication and mention
of the grant of the patent:
**28.01.2026 Bulletin 2026/05**

(21) Application number: **20794980.1**

(22) Date of filing: **24.04.2020**

(51) International Patent Classification (IPC):
**G01N 27/02** (2006.01)     **G01N 33/48** (2006.01)
**G01N 33/487** (2006.01)

(52) Cooperative Patent Classification (CPC):
**G01N 27/026; G01N 33/48728; G01N 33/5308**

(86) International application number:
**PCT/US2020/029766**

(87) International publication number:
**WO 2020/219842 (29.10.2020 Gazette 2020/44)**

(54) **METHOD AND DEVICE OF LABEL-FREE CHARACTERIZING OF NANOVESICLES BASED ON THEIR DIELECTRIC PROPERTIES**

VERFAHREN UND VORRICHTUNG ZUR MARKIERUNGSFREIEN CHARAKTERISIERUNG VON NANOVESIKELN AUF DER GRUNDLAGE IHRER DIELEKTRISCHEN EIGENSCHAFTEN

PROCÉDÉ ET DISPOSITIF DE CARACTÉRISATION SANS ÉTIQUETTE DE NANOVÉSICULES SUR LA BASE DE LEURS PROPRIÉTÉS DIÉLECTRIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.04.2019 US 201962838015 P**

(43) Date of publication of application:
**02.03.2022 Bulletin 2022/09**

(73) Proprietor: **University of Cincinnati
Cincinnati, OH 45221-0623 (US)**

(72) Inventor: **ESFANDIARI, Leyla
Cincinnati, OH 45208 (US)**

(74) Representative: **Plasseraud IP
104 Rue de Richelieu
CS92104
75080 Paris Cedex 02 (FR)**

(56) References cited:
WO-A1-2010/050898     WO-A1-2017/165267
WO-A1-2018/213562     WO-A1-2018/213562
US-A1- 2008 105 565     US-A1- 2009 092 989
US-A1- 2016 299 138     US-A1- 2018 276 339

- **MOHAMAD AHMAD SABRY ET AL: "A dielectrophoresis-impedance method for protein detection and analysis", AIP ADVANCES, AMERICAN INSTITUTE OF PHYSICS, 2 HUNTINGTON QUADRANGLE, MELVILLE, NY 11747, vol. 7, no. 1, 13 January 2017 (2017-01-13), XP012215300, DOI: 10.1063/1.4974290**
- **REDWOOD W R ET AL: "Dielectric studies on homogeneous phosphatidylcholine vesicles", BIOCHIMICA ET BIOPHYSICA ACTA, ELSEVIER, AMSTERDAM, NL, vol. 255, no. 2, 11 February 1972 (1972-02-11), pages 557 - 566, XP023505211, ISSN: 0005-2736, [retrieved on 19720211], DOI: 10.1016/0005-2736(72)90159-9**
- **WU LIQUN, LANRY YUNG LIN-YUE, LIM KIAN-MENG: "Dielectrophoretic capture voltage spectrum for measurement of dielectric properties and separation of cancer cells", BIOMICROFLUIDICS, vol. 6, 1 March 2012 (2012-03-01), pages 2, XP055755842, Retrieved from the Internet <URL:https://aip.scitation.org/doi/abs/10.1063/1.3690470> DOI: 10.1063/1.3690470**

**(Cont. next page)**

• SUGA KEISHI, TANABE TOMOYUKI, TOMITA HIBIKI, SHIMANOUCHI TOSHINORI, UMAKOSHI HIROSHI: "Conformational change of single-stranded RNAs induced by liposome binding", NUCLEIC ACIDS RESEARCH, vol. 39, no. Issue 20, 23 July 2011 (2011-07-23), pages 8891 - 8900, XP055755843, Retrieved from the Internet <URL:https://academic.oup.com/nar/article/39/20/8891/2409240> DOI: 10.1093/nar/gkr568

• GAWAD ET AL.: "Micromachined impedance spectroscopy flow cytometer for cell analysis and particle sizing", LAB CHIP, vol. 1, 13 August 2001 (2001-08-13), pages 76 - 82, XP008028057, Retrieved from the Internet <URL:https://pubs.rsc.org/no/content/articlehtml/2001/lc/b103933b> DOI: 10.1039/b103933b

**Description**

**CROSS REFERENCE TO RELATED APPLICATIONS**

This application claims priority to U.S. Provisional Application Serial No. 62/838,015, filed April 24, 2019.

**TECHNICAL FIELD**

The present invention relates to a method and device for characterizing nanovesicles. More specifically, it relates to characterizing the dielectric properties of exosomes.

**BACKGROUND OF THE INVENTION**

Electrical impedance sensing has been utilized as a label-free and non-invasive tool for screening viruses and pathogens, cellular response to infection, counting the nanoparticles suspended in solution, and characterization of size, cytoplasmic cargoes, and membrane capacitance of a single-cell. However, this technique has not yet been translated for characterization of extracellular vesicle exosomes, which are sub-micrometer in size.

Exosomes are small extracellular vesicles with diameters of ~40-150 nm, released from many cell types into the extracellular space. They are composed of a lipid bilayer membrane containing various receptors and tetraspanin proteins. They also encapsulate nucleic acids, proteins, and lipids in their lumen. Exosomes are promising biomarkers for several reasons: 1) they are highly abundant in all bodily fluids and therefore easily accessible; 2) their composition reflects their cellular origins and can therefore serve as indicators of pathology; and 3) they are stable. Also, it has been shown that exosomes secreted from different cellular origins, in particular pathogenic exosomes, undergo compositional changes and could have additional membrane receptors and/or elevated or suppressed levels of nucleic acids which can be associated with their total electric charges and dipoles. However, use of exosomes as biomarkers has been hampered by the lack of workable technologies to reliably isolate and rigorously characterize their unique properties in a timely manner. Although, some of the biophysical properties of exosomes such as size, density and morphology have been characterized before, their dielectric property which is associated with their unique compositional charges has not yet been investigated. US2008/105565 discloses a system for detecting analytes such as viral particles using impedance-based particle detection.

**SUMMARY OF THE INVENTION**

One embodiment of the present invention addresses this need by providing a means to characterize the small extracellular vesicles known as "exosomes" based on their unique dielectric properties. This characterization provides crucial information regarding the cell and tissue of their origin. Although the physical properties of exosomes such as size, density and shape have been studied before, their dielectric properties have not been investigated
In one embodiment of the present invention, a method of characterizing nanovesicles in accordance with claims 1-12 and 15 is disclosed. The method involves entrapping and sensing the dielectric properties of the nanovesicles using an electrical impedance sensing device. In another embodiment, the nanovesicles are selected from the group consisting of small extracellular vesicles, exosomes, liposomes, viruses and mixtures thereof. In another embodiment, the nanovesicles comprise exosomes.

In one embodiment, the nanovesicles are liposomes and they are characterized by distinguishing between liposomes with different membrane compositions. In another embodiment, the nanovesicles are liposomes and they are characterized by distinguishing between liposomes loaded with RNA and liposomes without RNA.

In another embodiment, the exosomes are characterized by distinguishing between exosomes secreted from different cellular origins. In one embodiment, the exosomes are characterized by distinguishing between exosomes with different size distribution but secreted from the same cellular origins. In another embodiment, the exosomes are characterized by distinguishing between exosomes with different cytosolic compositions.

In accordance with the invention, the electrical impedance sensing device comprises two or more electrodes that apply an AC field across the trapped nanovesicles in the range of from about 500 KHz to about 50 MHz. In another embodiment, the AC field is altered in magnitude and the results of the magnitude changes are analyzed to identify one or more biophysical dielectric properties of the exosomes. In yet another embodiment, the AC field is altered in phase and the results of the phase changes are analyzed to identify one or more dielectric properties of the exosomes. In one embodiment, the AC field is altered in magnitude and phase and the results of the magnitude and phase changes are analyzed to identify one or more dielectric properties of the exosomes. In another embodiment, the electrical impedance sensing device comprises an impedance analyzer, a power supply, a micromanipulator and a signal processor.

In another embodiment, the dielectric properties of the exosomes comprise opacity magnitude. In another embodiment, the two or more electrodes are placed at a distance from each other between about 20 μm and 100 μm.

In accordance with claims 13-15, a device for manipulating and analyzing particles in a suspending medium is disclosed. The device includes a first chamber configured to receive a back-fill medium; a second chamber configured to receive the suspending medium; a nanopipette including a first end located in the first chamber and a second end located in the second chamber, the first end including an inlet and the second end including a tip; a first trapping electrode located in the first chamber; a second trapping electrode located in the second chamber; a first sensing electrode located adjacent to the tip; a second sensing electrode located adjacent to the tip and opposing the first sensing electrode; a signal source including a first terminal electrically coupled to the first trapping electrode and a second terminal electrically coupled to the second trapping electrode, the signal source configured to output a reference signal on the first terminal and a bias signal on the second terminal, the reference signal and the bias signal defining an electrical signal having a characteristic that generates a potential well that traps the particles proximate to the tip of the nanopipette; and an impedance amplifier electrically coupled to the first sensing electrode and the second sensing electrode. The first and second sensing electrodes generate an AC field that interacts with the particles proximate to the tip of the nanopipette, producing an AC field signal, which is transmitted to a signal processor.

In another embodiment, the device also includes a micromanipulator configured to control the distance between the first sensing electrode and the second sensing electrode. In another embodiment, the device also includes a microscope.

## BRIEF DESCRIPTION OF THE DRAWINGS

The foregoing summary, as well as the following detailed description of preferred embodiments of the application, will be better understood when read in conjunction with the appended drawings.

FIG. 1 is a perspective view of a tip of a nanopipette including an opening.

FIG. 2 is a diagrammatic view of the tip of FIG. 1 showing the effects of electrokinetic forces on a negatively charged particle proximate to the opening when a positive voltage is applied across bias and reference electrodes.

FIG. 3 is a diagrammatic view of the tip of FIG. 1 showing the effects of electrokinetic forces on a negatively charged particle proximate to the opening when a negative voltage is applied across bias and reference electrodes.

FIG. 4 is an illustration of a nanopipette dielectrophoretic device for exosomes entrapment.

FIG. 5 is a diagrammatic view of a device for selectively trapping of particles using the electrokinetic forces of FIGS. 3 and 4.

FIG. 6 is a diagrammatic view of the tip of FIG. 2 showing a plurality of particles being trapped by a potential well under the influence of a positive electric field generated by the positive voltage. FIG. 6 also shows the integrated dielectrophoretic (DEP) impedance system.

FIG. 7A an illustration of a dielectrophoretic (DEP) trapping system and an impedance measurement system comprising an attached micromanipulator and a second set of electrodes.

FIG. 7B is an illustration of a DEP trapping system and a sensing system including a second set of electrodes for impedance measurement and an impedance analyzer.

FIG. 8 is a graph with images showing two concentrations of 100 nm liposomes captured at the tip of the pipette and showing the overlap of opacity magnitude.

FIG. 9 is a graphic depiction of opacity magnitude measurements of two concentrations of liposomes entrapped at the tip of a pipette.

FIG. 10 is a graph showing opacity magnitude for distinguishing purified exosomes and 100 nm artificial liposomes for a range of frequencies.

FIG. 11 is an illustration of the frequency-dependent dielectric response of a single-shelled particle.

FIG. 12A is an illustration of Foster and Schwan's simplified circuit model at a wide range of frequency spectrum (50 KHz to 50 MHz).

FIG. 12B is an illustration of a single-shell model.

FIG 13 is an illustration of two different 100 nm liposomes with different membrane compositions, the first liposome having a 1:10 ratio of cholesterol to lecithin and the second liposome having a 10:1 ratio of cholesterol to lecithin.

FIG 14 is a graph of the opacity measurements of two different 100 nm liposomes with different membrane compositions.

FIG 15 is a graph showing the theoretical impedance model of two types of liposomes with different membrane compositions.

FIG 16 is a boxplot of the magnitude opacity comparison of two sets of sample liposomes with different membrane compositions and the mixture of them.

FIG 17 is a series of graphs showing the opacity measurements at different frequencies between two different 100 nm liposomes with different membrane compositions.

FIG 18 is a graph showing the experimental impedance spectra of 1XPBS (control), polystyrene beads and EVs.

FIG 19 is a graph showing the theoretical impedance model of three conditions.

FIG 20A is an illustration showing the isolation of exosomes from hepatocytes under different culture conditions. FIGs 20B-F are boxplots of the magnitude opacity for control exosomes, PA-treated exosomes and PA+GW-treated exosomes under various applied AC fields.

FIG 21 shows the opacity magnitude for control exosomes, PA-treated exosomes and PA+GW-treated exosomes at a wide range of frequency.

FIG 22 is a pair of graphs showing opacity magnitudes of exosomes from hTERT mesenchymal stem cell exosomes and non-small cell lung cancer (NSCLC).

FIG 23 is a series of graphs showing opacity magnitudes of exosomes from hTERT mesenchymal stem cell exosomes and non-small cell lung cancer (NSCLC) under various applied AC fields.

FIG 24 is a series of graphs showing an opacity magnitude comparison and opacity magnitudes for exosomes from culture media of mouse primary hepatocytes: 1) Wild-type (GFP-) sample, and 2) Green fluorescent protein-transgenic (GFP+) sample.

FIG 25 is a series of graphs showing impedance measurements of liposomes with and without loaded tRNA.

FIG 26 is a series of graphs showing boxplots of magnitude opacity for liposomes without/with transfer RNA encapsulate inside under a wide range of frequencies.

FIG 27 is a chart showing the concentration of exosomes collected at four different size ranges for HUVEC and MDA-MB-231.

FIG 28 is a series of boxplots showing the magnitude opacity of exosomes with different size distribution collected from HUVEC under a wide frequency spectrum.

FIG 29 is a series of boxplots showing the magnitude opacity of exosomes collected with different size distribution from MDA-MB-231 under a wide frequency spectrum.

## DETAILED DESCRIPTION OF THE INVENTION

The details of one or more embodiments of the disclosed subject matter are set forth in this document. Modifications to embodiments described in this document, and other embodiments, will be evident to those of ordinary skill in the art after a study of the information provided herein.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. Also, in some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

While the following terms are believed to be well understood by one of ordinary skill in the art, definitions are set forth to facilitate explanation of the disclosed subject matter. Unless defined otherwise, all technical and scientific terms used herein have the same meaning as commonly understood by one of ordinary skill in the art to which the disclosed subject matter belongs.

As used herein, the term "about," when referring to a value or to an amount of mass, weight, time, volume, size, concentration or percentage is meant to encompass variations of in some embodiments $\pm 20\%$, in some embodiments $\pm 10\%$, in some embodiments $\pm 5\%$, in some embodiments $\pm 1\%$, in some embodiments $\pm 0.5\%$, and in some embodiments $\pm 0.1\%$ from the specified amount, as such variations are appropriate to perform the disclosed method.

The term "nanovesicle" refers to small (diameter between 20-250 nm) vesicles including the lipid bilayer membrane surrounding the interior aqueous space. "Small extracellular vesicles": "Exosome" means a sub-type of extracellular vesicle arising from the endosomal network and ranging in size from about 40 to 150 nm. The term "liposome" means a particle including lipid-containing molecules arranged to form a unilamellar or multilamellar membrane wall surrounding an interior volume. Many different viruses with similar size range and membrane composition can be characterized using the present invention, including, but not limited to, influenza viruses, coronavirus, adenovirus, and rhinovirus.

EXOSOME CHARACTERIZATION

An embodiment of the present invention involves trapping exosomes and then detecting and analyzing their dielectric properties. Since exosomes are charged particles and their physical compositions are similar to their cell of origin, by applying an AC field across the trapped exosomes, the propagated electric field distribution is altered in magnitude and phase depending on their unique membrane capacitance and cytosolic (inner lumen) conductance. This alteration has been investigated at a wide range of frequency spectrum (500 KHz to 50 MHz) to characterize exosomes based on their unique dielectric properties.

In an embodiment, the present invention incorporates a dielectrophoretic (DEP) nanopipette device that is capable of isolating biomolecules based on their surface charge and size in a buffer with high ionic concentration. The device can operate with DC voltage as low as 0.6 V/Cm, which is much lower than the conventional DC DEP methods (350 V/cm). Because a low voltage may be used, the isolated biomolecules maintain their integrity and functionality for further analysis. Also, in an embodiment, particles can be rapidly trapped in as little as 100 seconds and the sample volume may be as low as 50 $\mu$l. Furthermore, the DEP nanopipette device has a high spatial resolution allowing it to entrap secreted molecules near living cells.

In an embodiment, the DEP nanopipette device is a conical, glass nanopipette. In one embodiment, the glass is borosilicate glass. The surface of the nanopipette induces electroosmosis under applied DC voltage. For example, a borosolicate nanopipette has deprotonated Si-OH, which induces the electroosmosis. The diameter of the opening or pore of the nanopipette tip may be, for example, 500 nm, 1000 nm, or 2000 nm. Accordingly, the diameter of the pore may be in the range of 500 nm to 2000 nm.

In an aspect, the entrapment of molecules is charge selective and can be controlled by the polarity of the applied voltage. This is achieved by attracting the molecules with high surface charge to the nanopipette's tip by the dominant electrophoretic force and the molecules with low surface charge by the electroosmosis flow as the voltage polarity is reversed. The non-uniform electric field at the tip will induce a negative DEP force on molecules, which prevents them from entering into the pipette and accumulates them by the tip. The applied voltage may be, for example, less than 10 V/cm, less than 6 V/cm, less than 4 V/cm, and as low as 0.6 V/cm. Accordingly, the applied voltage may be in the range of 0.6 V/cm to 10 V/cm.

The entrapment of the molecules can be qualitatively and qualitatively measured by microscopic observation and conductance measurements across the pipette respectively. As molecules cluster by the tip, the conductance across the opening changes based on the size of the particle. The unique conductance change across the nanopipette indicates the size and the rigidity of the molecules. For further quantitative analysis, the entrapped molecules can be released into a second chamber containing low ionic solution by applying the reverse voltage polarity. At low ionic solutions, the high velocity outward fluid stream will push the molecules away from the pore and into the second chamber.

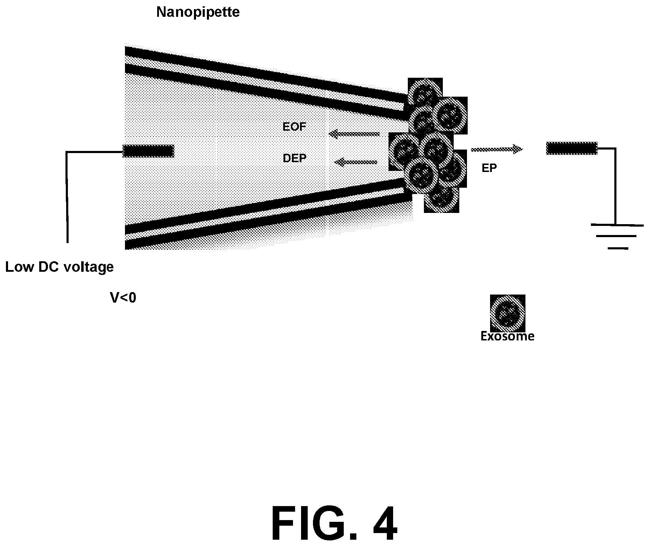

When voltage is applied across the glass nanopipette, particles in the suspending medium will be driven by three forces: electrophoresis (EP); dielectorphoresis (DEP); and electroosmosis (EOF) (see Figure 4). The balance of these forces can lead to trapping the particles. These forces can be calculated using a series of electrokinetic equations and modeled by COMSOL Multiphysics. The same analysis can be expanded for particles with lower surface charge and smaller size to evaluate the entrapment efficiency and selectivity in various experimental conditions such as different applied DC voltage, various salt concentration, and the diameters of the tip opening.

EXOSOME ENTRAPMENT

Embodiments of the invention incorporate a nanopipette device configured to rapidly trap particles using an electric field generated by a direct current electrical signal. Advantageously, the particles in a bulk suspending medium are trapped in a trapping zone or region proximate to the tip of the nanopipette, thus facilitating the collection of particles from bulk suspending mediums. Particles may be trapped in suspending mediums having various ionic strengths. Experimental results have been obtained using 510 nm carboxylic acid polystyrene (COOH-PS) beads to demonstrate the electrokinetic forces involved. These forces include electrophoretic, dielectrophoretic, and electro-osmotic forces. These results demonstrate a correlation between the induced electrokinetic forces and the number of trapped particles. Numerical modeling and empirical observations have been used to determine physical characteristics, such as the applied voltage, the ionic strength of the suspending medium, and the opening diameters necessary to generate potential wells that selectively trap particles within a desired trapping region.

Embodiments of the nanopipette device may use a low amplitude Direct Current (DC) electrical signal (e.g., DC voltage or current) to generate a potential well in a collection chamber containing a bulk suspension medium. The potential well may rapidly and selectively capture and quantify biological materials, such as microvesicles, near living cells with low concentration sensitivity and spatiotemporal resolution. These particles may be captured using significantly lower voltages as compared to conventional insulator-based dielectrophoresis devices. Utilizing a nanopipette formed from glass provides a simple and cost-effective fabrication procedure as compared to conventional insulator-based dielectrophoresis devices made using microfabrication techniques. Use of nanopipettes also allows the applied voltage to be reduced significantly due to the small conical geometry of the tip.

According to an embodiment of the invention, a device is presented that isolates biological materials, such as biomolecules, microvesicles, cells, and/or other particles, based on their surface charge and size in a buffer solution with a high ionic concentration. The device can operate with electric field strengths as low as 0.6 V/cm. This electric field strength is significantly lower than conventional DC dielectrophoresis methods, which typically require electric field strengths of at least 350 V/cm. Because a lower voltage may be used, the isolated biological particles may maintain their integrity and functionality for further analysis. Particles may be trapped in as little as 100 seconds and the sample volume may be as low as 50 μl. Furthermore, the dielectrophoresis nanopipette device has a high spatial resolution allowing it to trap secreted particles near living cells.

The device may include a glass nanopipette having a conical tip and may be formed from a suitable material, such as borosilicate glass. The surface of the nanopipette may induce electro-osmotic flow in response to application of a DC voltage. Borosilicate nanopipettes may include deprotonated Si-OH to induce the electro-osmotic flow. The diameter of the opening or pore of the nanopipette tip may be, for example, 500 nm, 1000 nm, or 2000 nm, Accordingly, the diameter of the opening may be in the range of 500 nm to 2000 nm, although embodiments of the invention are not limited to any particular range of opening sizes.

Trapping of certain particles may be charge selective, in which case the trapping can be controlled by the polarity of the applied voltage. For example, charge selectivity may result from particles with high surface charge being urged toward the tip of the nanopipette by a dominant electrophoretic force. In contrast, particles with a low surface charge may be urged toward the tip by a dominant electro-osmotic force, e.g., in response to the polarity of the voltage being reversed. The non-uniform electric field at the tip tends to induce a negative dielectrophoresis force on particles, which in some cases may prevent the particles from entering the nanopipette, causing the particles to accumulate on or proximate to the tip. The applied electric field strength may be, for example, less than 10 V/cm, less than 6 V/cm, less than 4 V/cm, and as low as 0.6 V/cm. Accordingly, the applied voltage may be sufficient to generate an electric field strength in the range of 0.6 V/cm to 10 V/cm, although embodiments of the invention are not limited to this range of field strengths.

When a voltage is applied across the glass nanopipette, the resulting electric field may act on particles in the suspending medium by way of three forces: an electrophoretic force, a dielectrophoretic force; and an electro-osmotic force that is due to an electro-osmotic flow of the suspending medium.

The electrophoretic, dielectrophoretic, and electro-osmotic forces may act on a charged particle with different potential polarities. Balancing these forces can lead to trapping of particles having certain characteristics, such as size, charge, or conductance. The forces can be calculated using a series of electrokinetic equations and modeled on a computer. The same analysis can be expanded for particles with different surface charge and/or size to evaluate the trapping efficiency and selectivity in various experimental conditions such as different electric field strengths and polarities, various ionic concentrations in the suspending medium, and geometric characteristics of the tip such as the size of the opening.

Embodiments of the present invention trap particles by generating a zero-net force region, or "potential well", that selectively traps particles by balancing the electrokinetic forces acting on the particles. These electrokinetic forces may include the dielectrophoretic force, the electrophoretic force, and drag between the particles and the flow of fluid caused by electro-osmosis (i.e., the electro-osmotic force) or pressure differentials. Particles trapped in the potential well may include liposomes and exosomes, which may be extracted directly from a bulk sample solution.

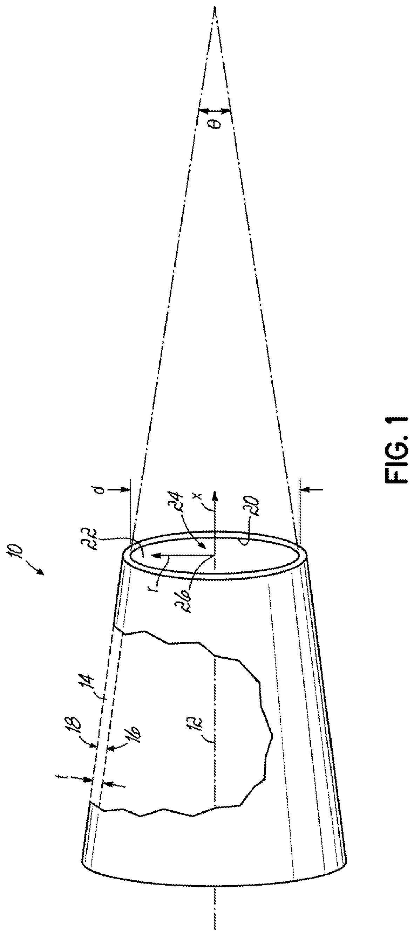

FIG. 1 depicts the tip 10 of a micropipette in accordance with an embodiment of the invention. The tip 10 may have a generally conical shape that is symmetrical about a central axis 12. The tip 10 includes a wall 14 having an inner surface 16 that defines an interior portion of the tip 10, an outer surface 18, and a thickness t. An edge 20 of inner surface 16 may define an opening 22 having a diameter d at the distal end of the tip 10. Opening 22 may include an interior side that faces toward the interior of tip 10, and an exterior side that faces away from the tip 10.

The central axis 12 of tip 10 may define a longitudinal axis x of a coordinate system 24. The coordinate system 24 may also include an origin 26 located at a point where the central axis 12 intersects a plane defined by opening 22, and a radial axis that is orthogonal to and intersects the longitudinal axis at the origin 26. The longitudinal and radial axes x, r of coordinate system 24 may be referred to herein to describe relative positions and/or orientations of forces acting on particles and/or locations of regions with respect to the opening 22. The inner and outer surfaces 16, 18 of tip 10 may be tapered at an angle Θ such that the diameter of the opening 22 is less than the diameter of the inner surface 16 at other points along the central axis 12 of tip 10.

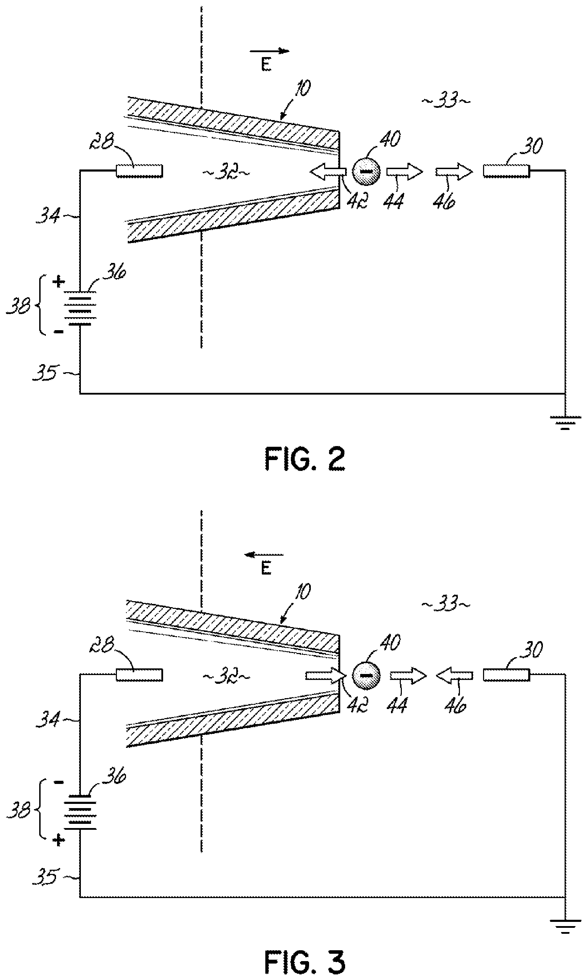

Referring now to FIGS. 2 and 3, an electric field may be generated in the region proximate to the tip 10 by applying an electric signal across a bias electrode 28 and a reference electrode 30. The bias electrode 28 may contact a back-fill medium 32 fluidically coupled to the opening 22 of tip 10 from the interior side of the opening 22, and the reference electrode 30 may contact a suspending medium 33 fluidically coupled to the opening 22 of tip 10 from the exterior side of opening 22. The bias electrode 28 and reference electrode 30 may be electrically coupled to a respective bias terminal 34 a respective reference terminal 35 of a signal source 36. The signal source 36 may be configured to generate an electrical signal 38 across the terminals 34, 35 so that a bias signal is applied to the bias electrode 28 and a reference signal is applied to the reference electrode 30. The electrical signal 38 may be a voltage (depicted) or a current having a constant or time-varying amplitude. Each of the electrodes 28, 30 may be electrically coupled to the opening 22 by their respective medium 32, 33.

The electrical signal 38 may produce electric fields proximate to the tip 10 that cause one or more forces to act on particles suspended in the suspending medium 33. By way of example, a particle 40 may be located proximate to the opening 22 (e.g., between 0 and 2000 nm from the opening 22) along the longitudinal axis on the exterior side of the opening 22. Forces acting on the particle 40 due to the electric fields proximate to the tip 10 may include an electrophoretic force 42, a dielectrophoretic force 44, and an electro-osmotic force 46.

The electrophoretic force 42 may result from an electrostatic phenomenon that causes electrically charged particles to be attracted toward an opposite charge and away from a like charge. The motion of particles relative to a liquid due to the influence of an electrophoretic force is known as "electrophoresis".

The dielectrophoretic force 44 may result from the effects of a nonuniform electric field on a particle. When a dielectric particle is exposed to a nonuniform electric field, the field may induce a dipole in the particle. Because the field is nonuniform, one end of the dipole may be in a region of the field having a higher strength than the other end of the dipole. This may cause the dipole to align with the field and to be urged in the direction of increasing field strength. The movement of particles in a liquid due to nonuniform electric fields is known as "dielectrophoresis".

The electro-osmotic force 46 may result from a flow of the media 32, 33 known as electro-osmosis. Electro-osmosis can be induced in a region of a liquid containing ions, such as a buffer solution, by introducing a voltage differential across the region, and is believed to be due to the movement of the ions in the liquid induced by the resulting electric field. Thus, the level of electro-osmosis in a liquid may depend in part on the number of ions present in the liquid.

As shown by FIG. 2, if the particle 40 is a negatively charged particle, and the electrical signal 38 causes the bias electrode 28 to have a positive voltage relative to the reference electrode 30, the electrophoretic force 42 may urge the particle 40 in a negative direction along the longitudinal axis x, i.e., toward the region of higher electric potential. In contrast, the dielectrophoretic force 44 and electro-osmotic force 46 caused by the positive electric field E generated by the positive voltage across electrodes 28, 30 may urge the particle 40 in a positive direction along the longitudinal axis x, i.e., toward the region of lower electric potential.

As shown by FIG. 3, if the particle 40 is a negatively charged particle, and the electrical signal 38 causes the bias electrode 28 to have a negative voltage relative to the reference electrode 30, the electrophoretic force 42 and dielectrophoretic force 44 may urge the charged particle 40 in a positive direction along the longitudinal axis x, i.e., toward the region of higher electric potential. In contrast, the electro-osmotic force 46 caused by the negative electric field E may urge the charged particle in a negative direction along the longitudinal axis x, i.e., toward the region of lower electric potential. Thus, reversing the electric field E reverses the directions of the electrophoretic force 42 and electro-osmotic force 46, but the direction of the dielectrophoretic force 44 remains positive. The electrophoretic, dielectrophoretic, and electro-osmotic forces can be controlled in several ways, including by adjusting the dimensions of the tip 10, the electrical signal applied to the electrodes 28, 30, and the ionic content of the medium in which the charged particle 40 is suspended.

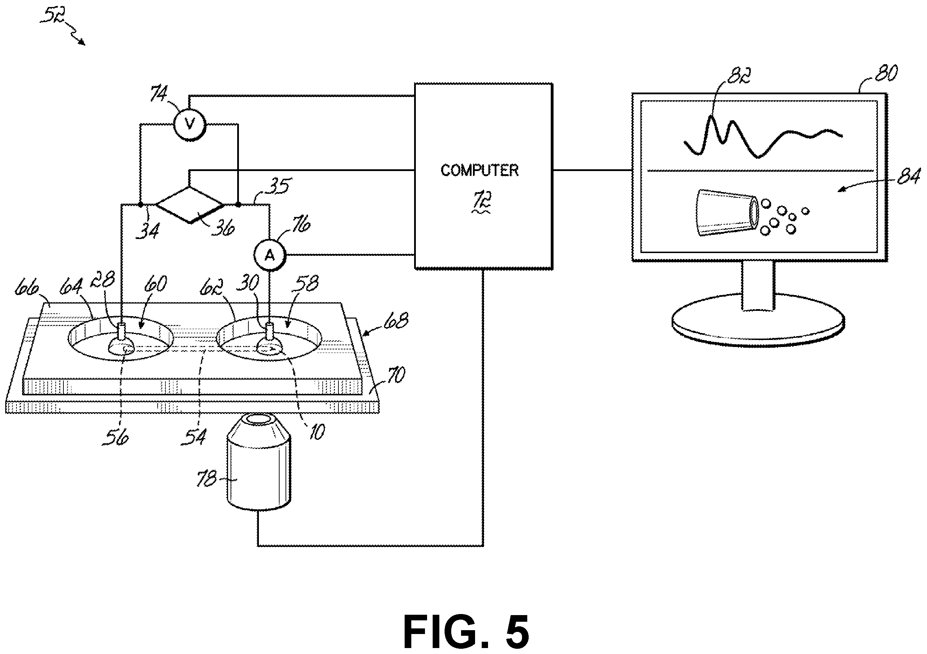

FIG. 5 depicts a device 52 in accordance with the present invention configured to selectively capture particles 40 using a potential well 50 in accordance with an embodiment of the invention. The device 52 includes a nanopipette 54 comprising the tip 10 at a distal end thereof and an inlet 56 at a proximal end thereof. The tip 10 of nanopipette 54 may be positioned in a collection chamber 58 configured to receive a suspending medium, and the inlet 56 of nanopipette 54 may be located in a back-fill chamber 60 or another reservoir configured to receive a back-fill medium. Each chamber 58, 60 may be defined, for example, by a respective aperture 62, 64 in a top sheet 66 that forms a side-wall of the respective chamber 58, 60 and a bottom sheet 68 having a top surface 70 that forms a bottom of the respective chamber 58, 60.

In an embodiment of the invention, the nanopipette 54 may be made from borosilicate, aluminosilicate, quartz, or another suitable material. The top sheet 66 may comprise a viscoelastic material, such as polydimethylsiloxane (PDMS), and the bottom sheet 68 may comprise a rigid optically transparent material, such as glass. The use of a viscoelastic material may allow the top sheet 66 to flow over and mold to the outer surface 18 of nanopipette 54 and/or a top surface 70 of bottom sheet 68. The top sheet 66 may thereby provide a liquid-tight seal against the nanopipette 54 and/or bottom sheet 68.

The device 52 may further include a computer 72 in communication with the signal source 36 and one or more sensors. The sensors may include a voltage meter 74 configured to measure the voltage provided to the electrodes 28, 30, a current meter 76 configured to measure the current flowing through the electrodes 28, 30 (and hence through the opening 22), and a camera 78 configured to capture on or more images (e.g., a series of images comprising a video stream) of the region proximate to the tip 10 of nanopipette 54, e.g., through bottom sheet 68. The computer 72 may be configured to control the amplitude of the electrical signal 38 output by the signal source 36, as well as capture data from each of the voltage meter 74, current meter 76, and camera 78. The computer 72 may present the voltage, current, and image data captured by the respective sensors on a display 80 in the form of one or more signal traces 82 and/or images 84 showing the movement of particles 40 in the collection chamber 58.

The electrophoretic force $F_{EP}$ acting on a spherical particle may be determined using the following equation:

$$F_{EP} = 6\pi\eta r \mu_{EP} E \quad Eqn. 1$$

where $\eta$ is the viscosity of the suspending medium, r is the radius of the particle, $\mu_{EP}$ is the electrophoresis mobility, and E is the applied electric field. The electrophoresis mobility $\mu_{EP}$ may be determined using the following equation:

$$\mu_{EP} = \frac{2\xi_p \varepsilon_m}{3\eta} \quad Eqn. 2$$

where $\xi_P$ is the zeta potential of the particle, and $\varepsilon_m$ is the permittivity of the suspending medium. The electrophoretic velocity $u_{EP}$ can be calculated from the electrophoretic mobility $\mu_{EP}$ using the following equation:

$$u_{EP} = \mu_{EP} E \quad Eqn. 3$$

The dielectrophoretic force $F_{DEP}$ acting on a spherical particle can be determined as:

$$F_{DEP} = 2\pi r^3 \varepsilon_m Re(f_{CM}) \nabla E^2 \quad Eqn. 4$$

where and $\nabla E^2$ is the electric field gradient, and $Re(f_{CM})$ is the Clausius-Mossotti factor, which is provided by:

$$Re(f_{CM}) = \frac{\varepsilon_p^* - \varepsilon_m^*}{\varepsilon_p^* + 2\varepsilon_m^*} \quad Eqn. 5$$

where $\varepsilon_p^*$ and $\varepsilon_m^*$ are the complex permittivity's of the particle and the medium, respectively. The complex permittivity may be expressed by $\varepsilon^* = \varepsilon - (j\sigma/\omega)$, where $\varepsilon$ is the real permittivity, $\sigma$ is the conductivity, and $\omega$ is the angular frequency of the applied electric field. The Clausius-Mossotti factor under DC field can also be represented as:

$$Re(f_{CM}) = \frac{\sigma_p - \sigma_m}{\sigma_p + 2\sigma_m} \quad Eqn. 6$$

where $\sigma_p$ is the conductivity of the particle and om is the conductivity of the suspending medium. Exemplary conductivities include $\sigma_p$ = 15.6 $\mu\beta/\alpha\eta$ for 510 nm COOH-PS beads, $\sigma_m$ =1.13 [$\mu$S/cm for deionized water, and $\sigma_m$ = 3000 $\mu$S/cm for 10 mM potassium chloride solution. The dielectrophoretic velocity $u_{DEP}$ may be determined using equation 7:

$$u_{DEP} = -\mu_{DEP} \nabla E^2 \quad Eqn. 7$$

where $u_{DEP}$ is the dielectrophoretic mobility. The dielectrophoretic mobility can be determined using equation 8:

$$\mu_{DEP} = \frac{r^2 Re(f_{cm})\varepsilon_m}{3\eta} \quad Eqn.\,8$$

The electro-osmotic force $F_{EOF}$ is may be determined using Equation 9, and (absent any flow other than electro-osmotic flow) is equal to the drag force $F_{drag}$.

$$F_{EOF} = F_{drag} = \frac{1}{2} C_d \rho v^2 A \quad Eqn.\,9$$

where $C_d$ is the coefficient of drag for the particle, $\rho$ is the density of suspending medium, v is the velocity of fluidic flow relative to the particle, and A is the cross-sectional area of particle. The electro-osmotic flow velocity $u_{EOF}$ may be determined as:

$$u_{EOF} = \mu_{EOF} E = \frac{\varepsilon E \zeta}{4\pi\eta} \quad Eqn.\,10$$

Where $\mu_{EOF}$ is the electro-osmotic flow mobility, $\varepsilon$ is the permittivity of the suspending medium, and $\zeta$ is the zeta potential of the wall 14 of the nanopipette 54. The zeta potential for the glass nanopipette can be estimated using Graham's equation, which relates the zeta potential to the estimated surface charge density of the micropipette.

The velocity of a negatively charged particle may be determined by the superposition of the flow of the surrounding bulk medium caused by electro-osmotic flow, the electrophoretic velocity of a particle $V_{EP}$, and the dielectrophoretic velocity of a particle $V_{DEP}$. Adding each of these effects may allow the particle velocity to be determined using Eq. 11:

$$v = u + \mu_{EP} E - \mu_{DEP} \nabla E^2 \quad Eqn.\,11$$

To quantify the magnitude and direction of the velocity of the particles, a series of equations is solved below. The bulk fluid flow and the electric field E can be evaluated by solving the coupled system of electrokinetic equations- Poisson's equation, Nernst-Planck equation, and Stoke's equation. The electric field E through the electrostatic potential ($\phi$) can be described by Poisson's equation:

$$\nabla\phi(r) = -\frac{\rho_e(r)}{\varepsilon_0 \varepsilon_r} \quad Eqn.\,12$$

where $\varepsilon_0$ is the permittivity of free space (about $8.85\times10^{-12}$ F/m) and $\varepsilon_r$ is the relative permittivity of the material.

The flux of two ionic species (e.g., K+ and Cl-) may be defined using the Nerst-Planck equation:

$$J = -\left\{ D\nabla c - uc + \frac{Dze}{k_B T} c \left( \nabla\phi + \frac{\partial_A}{\partial_t} \right) \right\} \quad Eqn.\,13$$

where $D$ is the diffusivity, $c$ is the ionic concentration, $z$ is the valence of the ionic species, $e$ is the elementary charge, $k_B$ is the Boltzmann constant, $T$ is the temperature and $u$ is the velocity of fluid.

The relation between fluid velocity body force ($\rho_e(r)\nabla\phi(r)$) and the pressure gradient (Vp) is defined by Stoke's equation:

$$\eta \nabla^2 u = \rho_e(r)\nabla\phi(r) + \nabla p \quad Eqn.\ 14$$

For the simulations used to produce the below experimental results, $\eta = 1 \times 10^{-3}$ Pa-s, and Vp is 0.

SIMULATION METHODOLOGY

Simulation results were obtained using COMSOL® Multiphysics version 5.2a finite element analysis software, which can be obtained from COMSOL Inc. of Stockholm, Sweden, and are based on equations 1-14 above. The software was used to determine the distribution of electrophoretic, dielectrophoretic, and electro-osmotic forces acting on the particles based on factors including one or more of the characteristics of the electric signal, the suspending medium, the particles, and the tip.

The system being modeled comprised a 1-D model where Poisson's and Nernst-Planck Equations were solved for variations in the electric fields and concentration distribution of ionic species along the boundary of the system. The 1-D model served as the Dirichlet boundary conditions for the 2-D model, thereby emulating the nanopipette setup. A 2-D axisymmetric design comprising the borosilicate nanopipette suspended in a circular reservoir filled with monovalent buffered salt was constructed, and boundary conditions applied corresponding to the solution obtained from the Poisson-Boltzmann equation for electric potential. The conditions established that the electric potential did not diverge and the gradient of this potential on the nanopipette surface varied with the change in surface charge density.

The model computed a combination of multiple physical phenomena pertaining to different aspects of the system. Electrostatics catered to the surface charge and voltage related analysis, creeping flow was solved for the study of incompressible and non-isothermal flow along the glass walls of the nanopipette, and transport of diluted species was incorporated for the migration of ionic species with the applied fields. The solution of the system provided the electric field and gradient of the square of electric field along the entire nanopipette length.

CHARACTERIZATION SYSTEM DESIGN

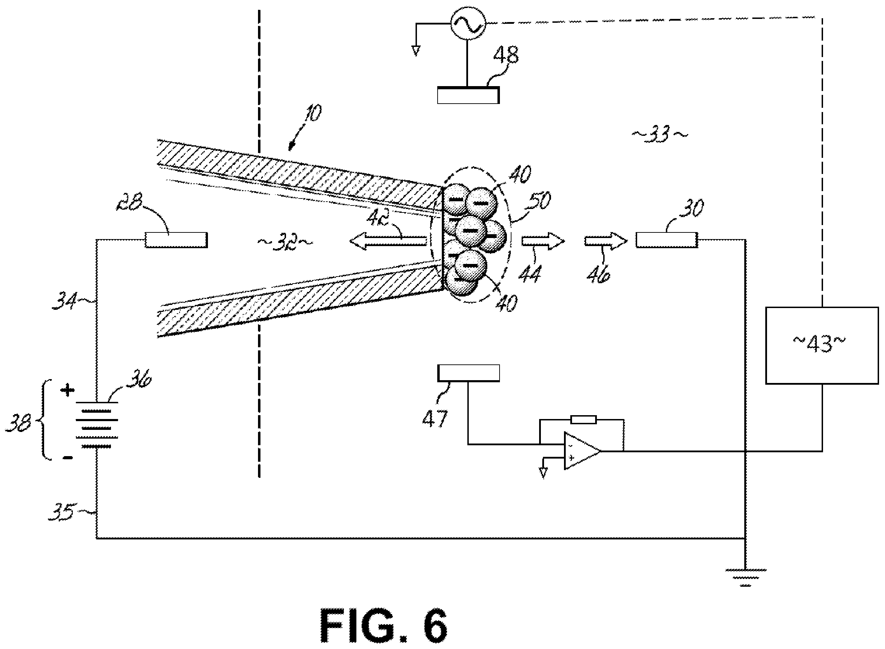

FIG. 6 depicts a potential well 50 comprising a region of zero or near-zero net force in which electrophoretic force 42, dielectrophoretic force 44, and electro-osmotic force 46 acting on the particles 40 essentially cancel each other out. Outside of the potential well 50, the net effect of the electrophoretic, dielectrophoretic, and electro-osmotic forces acting on the particles 40 may urge the particles 40 toward the potential well 50, thereby forming a trapping region. The potential well 50 may be produced proximate to the opening 22 of tip 10 in response to the application of an electric signal to the electrodes 28, 30. The characteristics of the potential well 50 (e.g., shape, size, volume, and location) may depend at least in part on the characteristics of the electric signal (e.g., the amplitude and polarity), the characteristics of the tip 10 (e.g., the taper angle Θ and diameter d of opening 22), the characteristics of the medium 32 in the nanopipette and/or the medium 33 in which the particles are suspended (e.g., the ionic concentration and/or the viscosity of each medium), and the characteristics of the particles 40 (e.g., size and charge). An impedance analyzer 43 is connected to a first sensing electrode 47 and a second sensing electrode 48. The sensing electrodes 47 and 48 apply an AC field across the trapped particles 40. The signal resulting from this AC field is sent to a signal processor (not shown). When the propagated electric field distribution is altered in magnitude and/or phase the signal processor enables characterization of the particles 40 depending on their unique membrane capacitance and cytosolic (inner lumen) conductance.

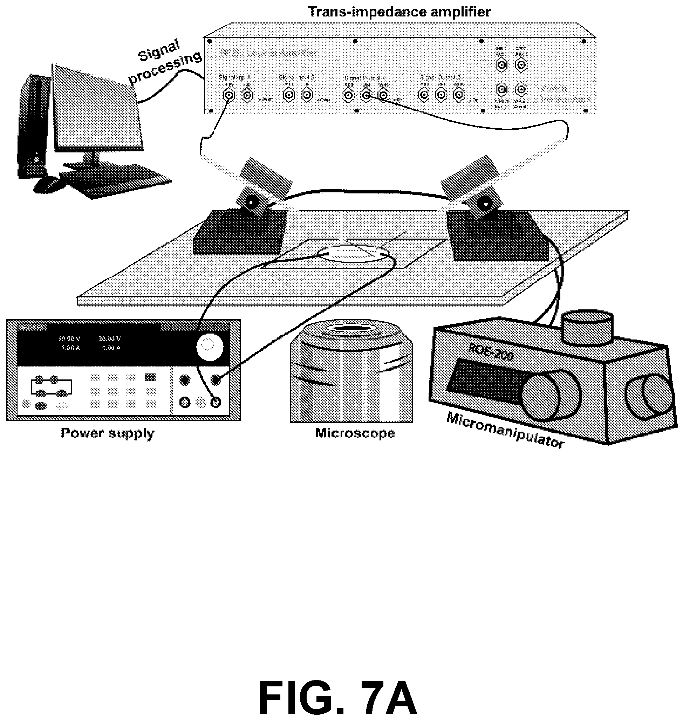

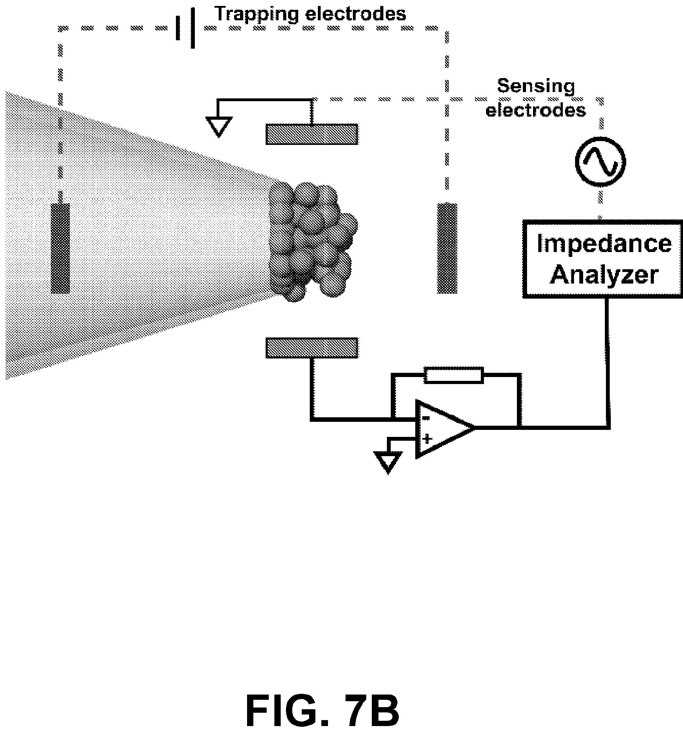

Figure 7A is an illustration of the impedance measurement setup. The diagram is not to scale. Figure 7B is a schematic of the insulator-based nanopipette dielectrophoretic device. The particles were immobilized at the tip of the pipette under the applied DC field via the power supply. The impedance of the particles was measured and digitized by a trans-impedance amplifier.

CONVENTIONAL CHARACTERIZATION METHODS

Conventional characterization methods for exosomes include proteomic profiling and genomic detection. Proteomic profiling techniques include enzyme-linked immunosorbent assay (ELISA), western blot (WB), flow cytometry and chromatography. Examples of genomic detection are qRT-PCR, microarrays, and second-generation sequencing. However, these conventional methods break the structure of the exosomes in the labelling and lysing steps. The present invention allows analysis of exosomes while keeping them intact. This technique may lead to new personalized medicine and therapeutics.

IMPEDANCE CYTOMETRY BACKGROUND AND THEORETICAL MODEL

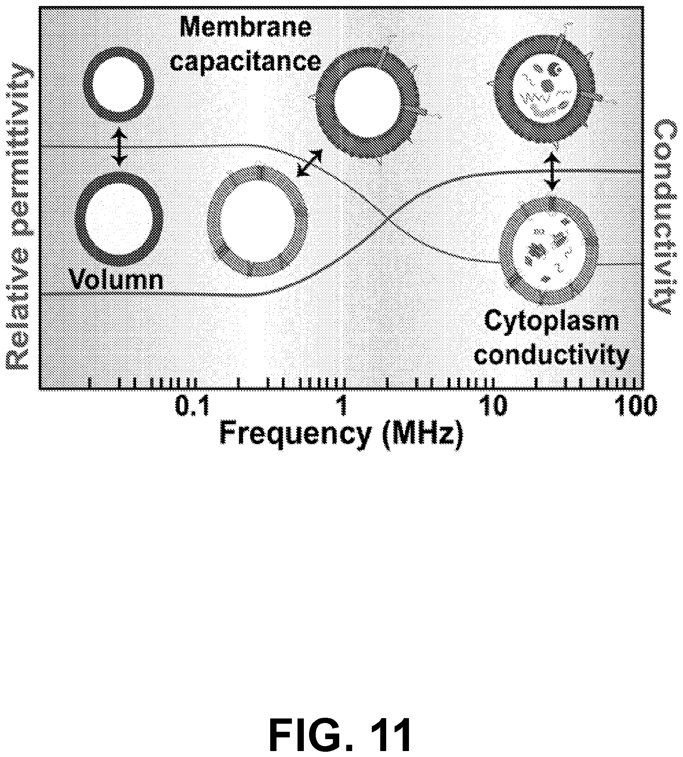

Maxwell's mixing theory is applied to analyze the dielectric behavior of cells in suspension under an AC voltage at varied frequencies (Figure 11). This figure is an illustration of the frequency-dependent dielectric response of a single-shelled particle. While not being bound by theory, we hypothesize that the dielectric properties of exosomal membrane and cytosol are similar to their cell of origin since their morphology mimics the cell of origin.

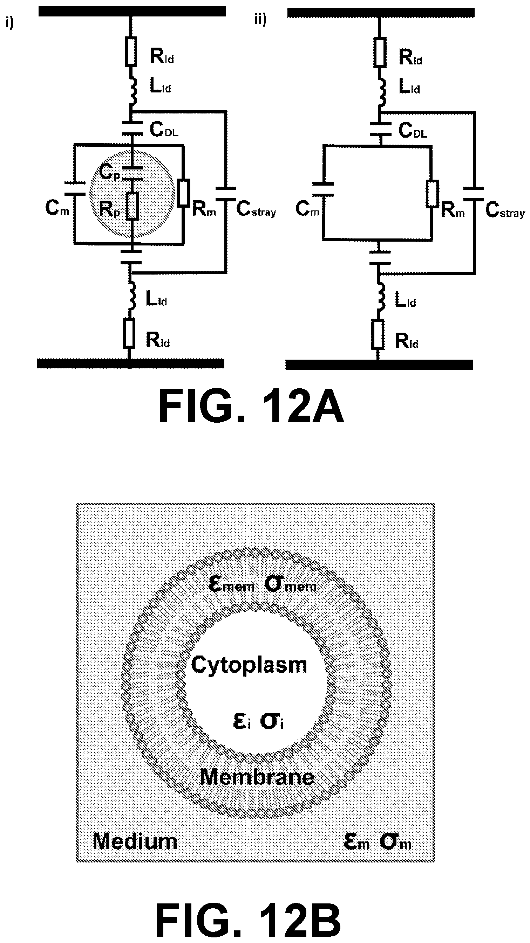

The dielectric properties of cells presenting in an AC field exhibit frequency dependent characteristics based on their impedance measurement. A simplified circuit model for a cell in suspension was developed by Foster and Schwan. Figure. 12A is an illustration of this circuit model. It is an equivalent circuit for modeling the impedance measurement system i) without and ii) with a vesicle. A single-shell vesicle in suspension is modeled as a capacitor $(C_p)$ which represents the membrane and a resistor $(R_p)$ which represents the cytoplasm in series based on the Foster and Schwan's simplified circuit model. The same model was utilized for the present invention to simulate the liposomes and EVs due to their similar structure of a lipophilic shell and an aqueous core as cell. An equivalent circuit was constructed to model the impedance signal of entrapped particles 40 at the tip 10 of the micropipette as shown in Figure 6. The inductance effect $(L_{ld}$ and $R_{ld})$ was introduced by the leading cables for the electrical connection between the electrodes and impedance analyzer was connected in series with the circuit. It was measured by connecting the cable with known terminations: open circuit, short line and resistor load. A double-layer capacitance $(C_{dl})$ was presented in the electrode-electrolyte interface due to the electrode polarization. The stray capacitance $(C_{stray})$ was introduced owing to the stored opposite electric charges on two electrodes under the electric field. $C_{stray}$ and $C_{dl}$ were estimated by measuring the impedance of electrolyte solutions with known conductivities, followed by fitting into the combination of constant phase element and Cole-Cole model. The number of the trapped nanovesicles was estimated by division of the approximated cluster of nanovesicles total volume by the volume of single vesicle. The estimation concentration of entrapped nanovesicles was verified by collecting the entrapped vesicles in a fresh solution by reversing the DC voltage polarity, followed by NTA analysis. The cluster of entrapped vesicles was approximated as a capacitor connected with a resistor in series which represents the accumulated membrane and cytoplasm of the vesicles. Eqn. 15 provides the impedance of particles suspending in the medium based on the complex permittivity:

$$Z_{mix} = \frac{1}{j\omega\tilde{\varepsilon}_{mix}G} \qquad Eqn.\ 15$$

$$\tilde{\varepsilon}_{mix} = \tilde{\varepsilon}_m \frac{1+2\Phi\tilde{f}_{CM}}{1-\Phi\tilde{f}_{CM}}, \text{ with } \tilde{f}_{CM} = \frac{\tilde{\varepsilon}_{exo}-\tilde{\varepsilon}_m}{\tilde{\varepsilon}_{exo}+2\tilde{\varepsilon}_m} \qquad Eqn.\ 16$$

$$\tilde{\varepsilon}_{exo} = \tilde{\varepsilon}_{mem} \frac{\gamma^3 + 2\left(\frac{\tilde{\varepsilon}_i - \tilde{\varepsilon}_{mem}}{\tilde{\varepsilon}_i + 2\tilde{\varepsilon}_{mem}}\right)}{\gamma^3 - \left(\frac{\tilde{\varepsilon}_i - \tilde{\varepsilon}_{mem}}{\tilde{\varepsilon}_i + 2\tilde{\varepsilon}_{mem}}\right)} \qquad Eqn.\ 17$$

$$\tilde{\varepsilon} = \varepsilon - j\frac{\sigma}{\omega} \qquad Eqn.\ 18$$

in which $G_f$ represents the geometric constant, calculating as the ratio of electrode area to the electrodes gap A/g (m) for an idea parallel plate electrode system. $\tilde{\varepsilon}_{mix}$ is the complex permittivity of particles suspended in the medium which described by Maxwell's mixing theory which uses shell models (see Figure 12B) to simulate the dielectric properties of particles in suspension. $\omega$ is the angular frequency and $j^2 = -1$. $\tilde{f}_{CM}$ is the Clausius-Mossotti and $\phi$ is the volume fraction. $\tilde{\varepsilon}_p$ and $\tilde{\varepsilon}_m$ represent the complex permittivity of the particle and medium separately. Figure 12B is a diagram of a single vesicle in suspension. $\varepsilon_m$ and $\sigma_m$ represent the permittivity and conductivity of the medium; $\varepsilon_{mem}$ and $\sigma_{mem}$ depict the permittivity and conductivity of the membrane; $\varepsilon_i$ and $\sigma_i$ describe the permittivity and conductivity of the cytoplasm.

IMPEDANCE MEASUREMENT

In one embodiment, the present invention discloses an electronic impedance device to characterize the dielectric properties of multiple stationary exosomes. Exosomes are immobilized using a nanopipette dielectrophoretic device for exosomes entrapment, such as the one illustrated in Figure 3. Once multiple exosomes are immobilized at the tip of the micropipette utilizing dielectrophoretic (DEP) force under applied DC field, impedance measurements are taken. In accordance with the invention, impedance measurements are taken by applying 0.2Vpp sweeping from 0.5MHz to 50MHz to cover the particle size, membrane capacitance and interior conductance.

At low frequency, the interfacial polarization at the microelectrode surface (double-layer capacitance between the electrode and suspension) can be approximated as a capacitance in series with the measurement sample, which causes a reduction in signal-to-noise ratio. 0.5MHz is chosen as the lowest frequency to be used in the experiment as a compromise between the need for a frequency low enough to detect the size and yet high enough to guarantee a good signal-to-noise. At high frequency range, the stray capacitances in parallel with the measurement sample will shunt the particle impedance and affect the device sensitivity.

The magnitude opacity rules out the number of particles effect and thus the impedance measurements result in the dielectric properties of the average population of the exosomes. Opacity Magnitude is the ratio of impedance magnitude at high frequency (>1MHz) to the low frequency (e.g. 500kHz):

$$Opacity\ Magnitude = \frac{|Z_{high\,f}|}{|Z_{low\,f}|} \qquad Eqn.\ 19$$

EXAMPLES

Example 1

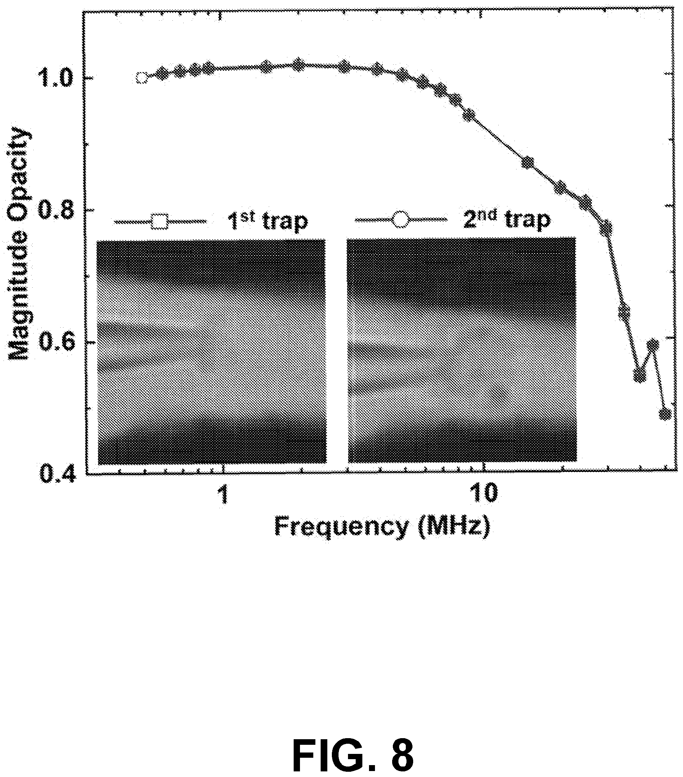

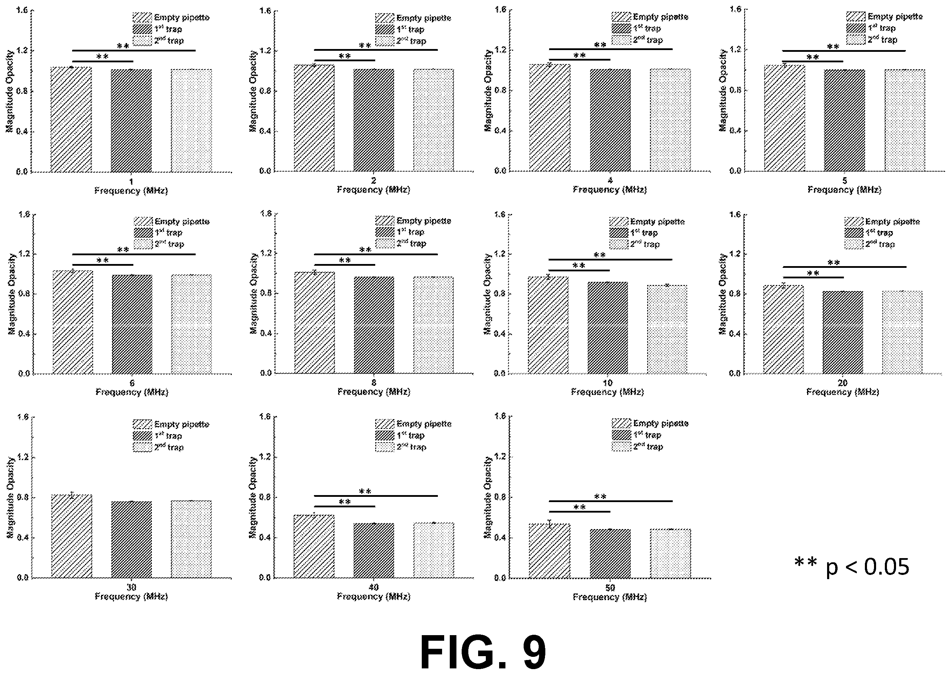

An electrical impedance sensing device that is integrated with a micropipette-DEP trapping device was developed to demonstrate the capability of the present invention to measure the impedance of the trapped particles. Prior to the entrapment, the impedance of the reservoir containing 10μL 1x PBS solution and the pipette tip was measured by placing two platinum electrodes with 100 μm diameters across the particle. The electrodes were positioned ~20 μm from one another using the MPC-325 micromanipulators (Sutter Instruments) under an inverted microscope (Nikon Eclipse TE2000-E). A 0.2Vpp sinusoidal voltage at frequencies sweeping from 500kHz to 50MHz was applied via a digital impedance analyzer (HF2LI, Zurich Instrument) and the output signal was retrieved with a trans-impedance amplifier (OPA111) and digital lock-in amplifier analyzer (HF2LI, Zurich Instrument). Each measurement was repeated three times and the impedance was plotted as a function of frequency to establish the baseline. Further, artificial liposomes with 100 nm diameters from 10μL 1x PBS solution containing ~ 109 particles/mL were trapped by applying the DC field (10 V/cm) across a 1μm pipette. The DC field was turned off after 5 minutes and the microscopic observations showed that the trapped particle remained stationary at the tip. The impedance measurements of the trapped liposomes were conducted three times followed by repeating the entrapment for 5 more minutes and impedance measurements. The magnitude and phase of the impedance measurements were plotted separately as a function of frequency and to rule out the effect of number of entrapped particles we calculated the opacity for each measurement for a range of frequencies. The opacity is the ratio of the high frequency to the lower frequency (~ 500 kHZ) which provides a parameter that is independent of the particles size and reflects the changes of membrane capacitance or cytosolic conductance. At the low frequency (<1MHz), the impedance measurement is dominated by the electric double layer, and the impedance value presents the size of the "cell". At the frequencies ranging from 1MHz to 10MHz, the cell membrane capacitance dominates the impedance value and as the frequency increases to > 10MHz the cytosolic conductance become dominant. The images of Figure 8 show the number of 100nm liposomes captured at two entrapments Figure 9 shows the opacity measurements of the two entrapments Specifically, it is an opacity magnitude comparison of liposomes trapped at different times (before the trap, 1st trap: 2 mins, 2nd trap:5 mins). The diameter of the micropipette was 1μm, and the distance between the two impedance measurement electrodes was 18μm. This data shows the independence of our device on the number of trapped particles.

Example 2

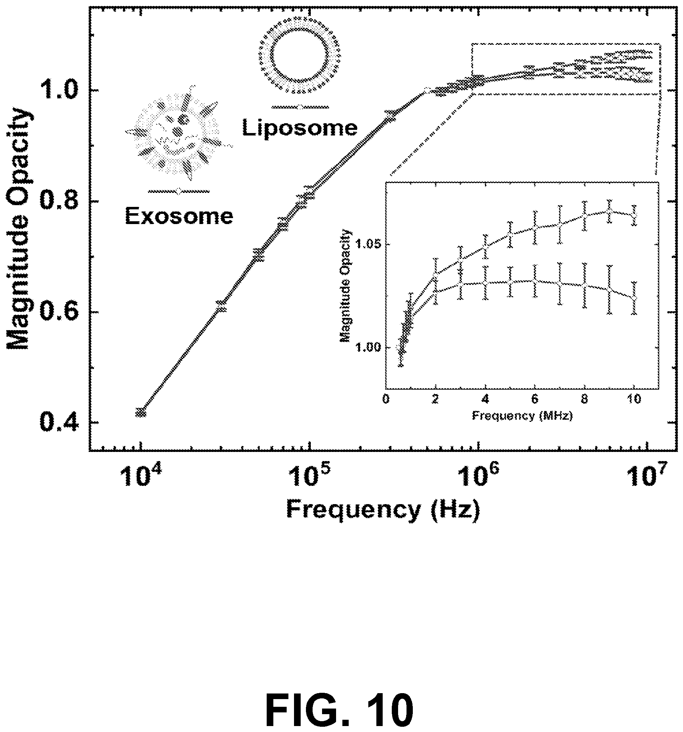

To demonstrate the sensitivity of the impedance sensor, two separate experiments were conducted utilizing purified exosomes (~100 nm) and the artificial liposomes as the same concentration of the particles were diluted in the chambers. The particles were trapped by DC field for 5 minutes followed by the impedance measurements. The data was extracted and the opacity was calculated for a range of frequencies. The results indicate that the opacity magnitude of both particles are the same at lower frequencies. However, in the range of 1MHz to 50 MHz, the opacity of the two particles were distinguishable due to the effect of their membrane capacitance (Figure 10). Exosomes have tetraspanin proteins embedded in their membrane and thus, its capacitance is different than the lipid bilayer construct in liposomes. These results show the capability of the system of the present invention to distinguish between the physical compositions of two nanovesicles of similar size.

## Example 3

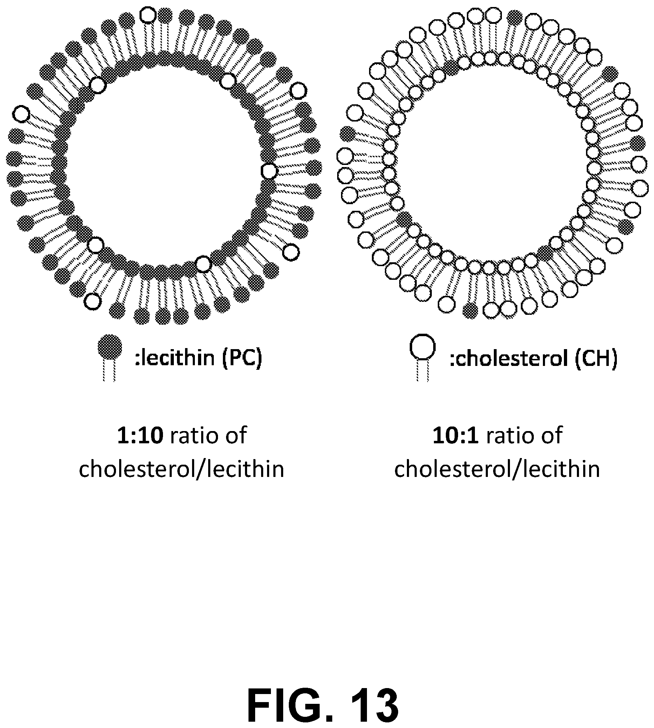

The effect of membrane dielectric properties was analyzed by constructing two different 100 nm liposomes with different membrane compositions (Figure 13). A first liposome had a 1:10 ratio of cholesterol to lecithin. This liposome had a membrane capacitance of $C_{LE}$=0.38 $\mu$F/cm$^2$ and resistance of $R_{LE}$ = 1.44$\times$10$^4$ $\Omega$/cm$^2$. The second liposome had a 10:1 ratio of cholesterol to lecithin. This liposome had a membrane capacitance of $C_{CH}$ = 0.61 $\mu$F/cm$^2$ and resistance of $R_{CH}$ =2.12$\times$ 10$^6$ $\Omega$/cm$^2$. The surface area of each 100 nm liposome was calculated: S = $\pi r^2$ = 7.9 $\times$ 10$^{-11}$cm$^2$.

The capacitance of the first liposome (1:10 ratio of cholesterol/lecithin) was

$$C_{1:10} = C_{CH} * \frac{1}{11}S + C_{LE} * \frac{10}{11}S = 3.14 \times 10^{-17}F$$

. The capacitance of the second liposome (10:1 ratio of cholesterol/lecithin) was

$$C_{10:1} = C_{CH} * \frac{10}{11}S + C_{LE} * \frac{1}{11}S = 4.19 \times 10^{-17}F$$

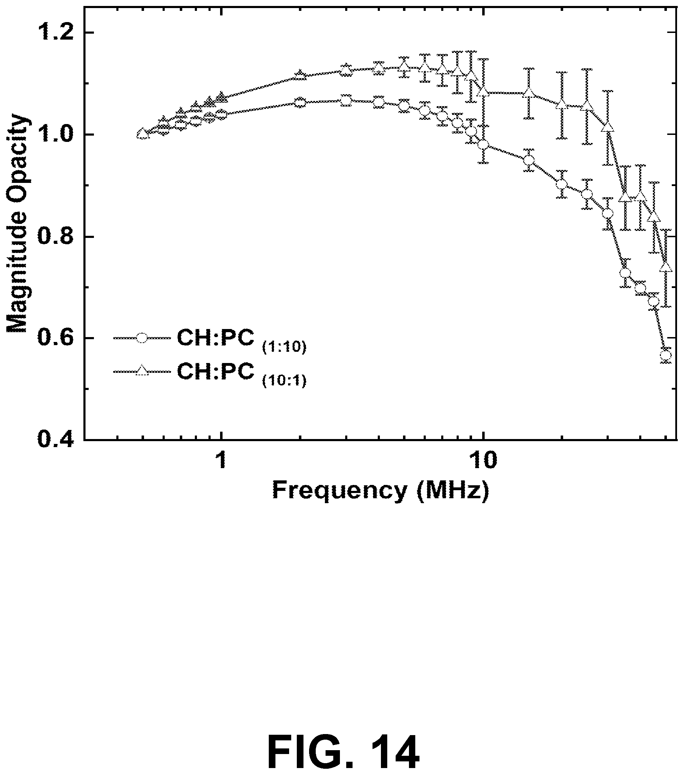

. Therefore, $C_{mem/1:10}$ < $C_{mem/10:1}$ (See Figure 14).

$$Z = R_{in} * \left(\frac{V_{in}}{V_{amp}} - 1\right) - R_{out} \qquad Eqn.\, 20$$

$$Z = \frac{1}{jwC_{mem}} \qquad Eqn.\, 21$$

$$V_{amp} \propto OM \qquad Eqn.\, 22$$

Where $C_{mem}$ is membrane capacitance, w is angular frequency, $R_{in}$ and $R_{out}$ = 50$\Omega$, and $V_{in}$ is the magnitude of the applied voltage (0.1V). $C_{DL}$ is double layer capacitance, $C_m$ is medium capacitance and $R_m$ is medium resistance. When $C_{mem}$ increases, Z will decrease. As a result, the measured $V_{amp}$ and opacity magnitude (OM) will increase.

$$C_{mem/1:10} < C_{mem/10:1}$$
$$\downarrow \qquad\qquad Eqn.\, 23$$
$$OM_{mem/1:10} < OM_{mem/10:1}$$

$$\widehat{Z} = R_{in} * \left(\frac{V_{in}}{V_{amp}} - 1\right) - R_{out}$$

$$Z = \frac{1}{j\widehat{\omega}C_{mem}} + j\widehat{w}R_{cyto} \qquad Eqn.\, 24$$

$$\omega = 2\pi f$$

$$V_{amp} \propto OM$$

Where $C_{mem}$ is membrane capacitance, $R_{cyto}$ is cytosol resistance.

$$\Delta V_{amp} \propto j * 2\pi f * (\Delta C_{mem}) \qquad Eqn.\, 25$$

At higher frequency range w, $\Delta V_{amp}$ will be higher. The difference of OM between two liposomes will be larger.

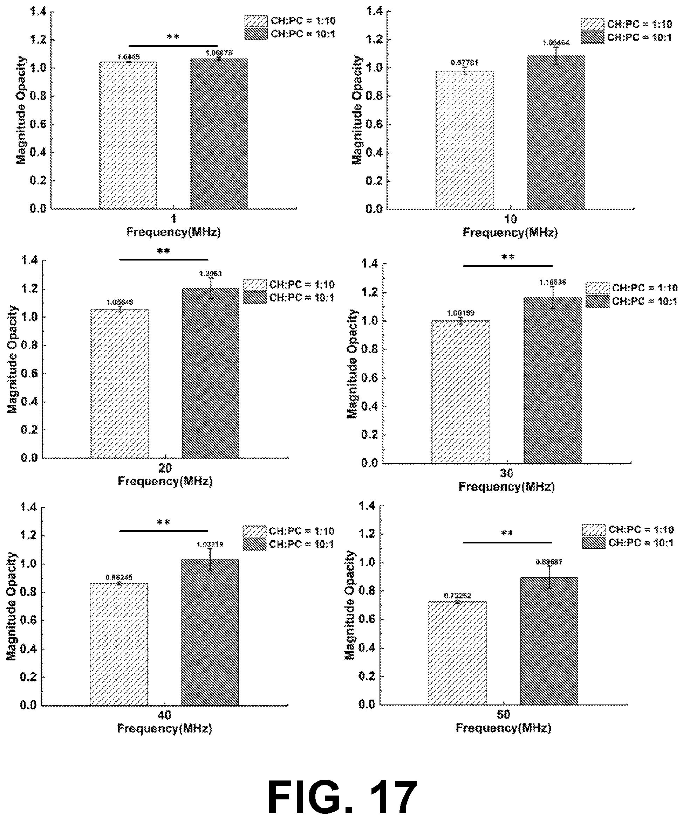

At the frequency range higher than 20MHz, the dielectric properties of the membrane start to affect the impedance measurement result. The opacity of two liposomes is significantly different from each other. Higher opacity is linked to the liposomes with high membrane capacitance (theoretical derivation is presented in Figure 17).

## Example 4

A magnitude opacity comparison was conducted between two liposomes with different membrane composition;

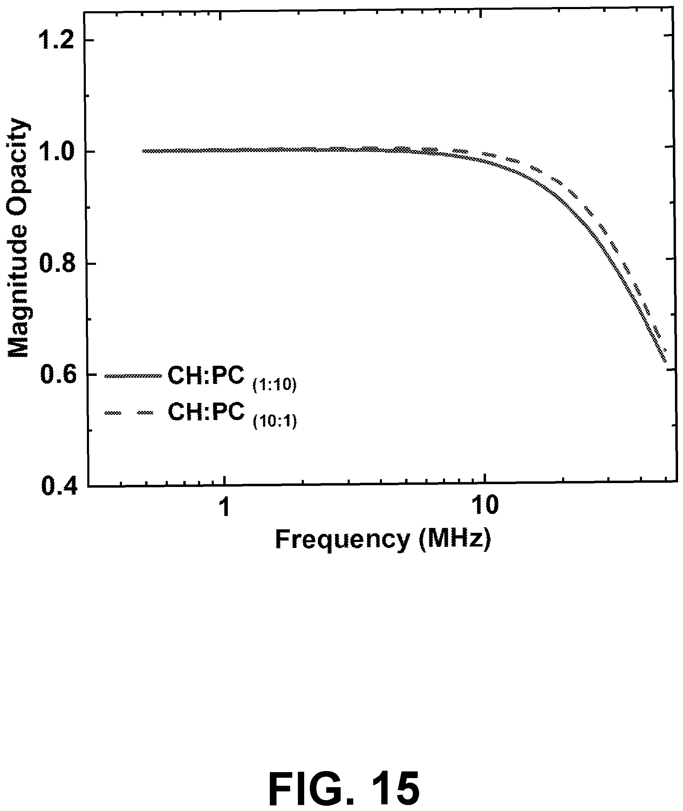

and their mixture. Results showed that the system is capable of distinguishing between liposomes with different membrane compositions and the mixture of them. Figure 15 shows the theoretical impedance model of two types of liposomes.

Example 5

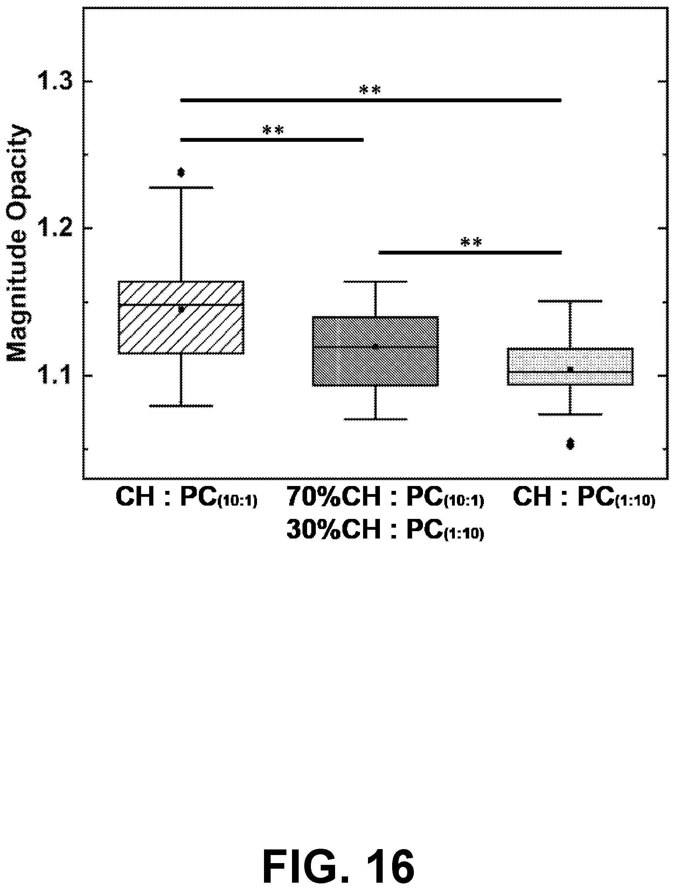

Figure 16 is a boxplot of the magnitude opacity comparison of three sets of sample liposomes: 1) CH:PC$_{(10:1)}$, 2) a mixture of 70%CH:PC$_{(10:1)}$ and 30%CH:PC$_{(1:10)}$, and 3) CH:PC$_{(1:10)}$. Approximately 75 sets of data were analyzed on each condition. **$p<0.05.$*

Example 6

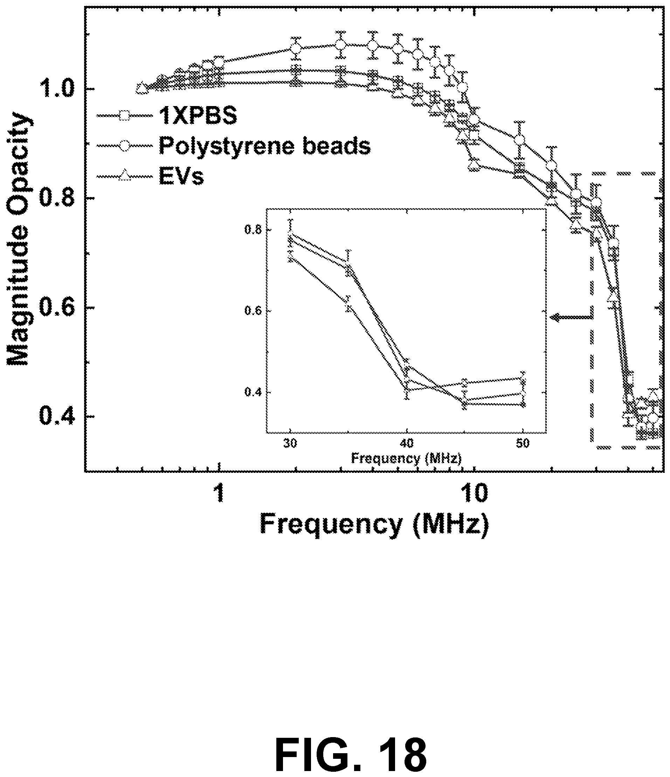

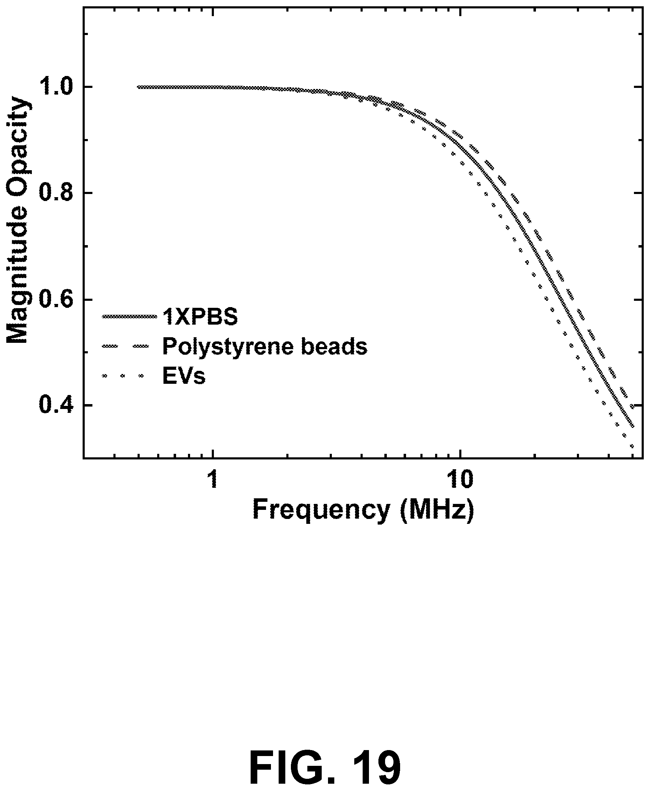

A magnitude opacity comparison was conducted among 1) electrolyte solution only (1x PBS), 2) 100 nm polystyrene beads and 3) exosomes (commercially purchased). The results showed that the experimental trend followed the developed theoretical model. Figure 18 shows the experimental impedance spectra of 1XPBS (control), polystyrene beads and EVs. The inset graph of FIG 18 is a zoom-in image of impedance spectrum between 30MHz and 50MHz. Figure 19 shows the theoretical impedance model of three conditions.

Example 7

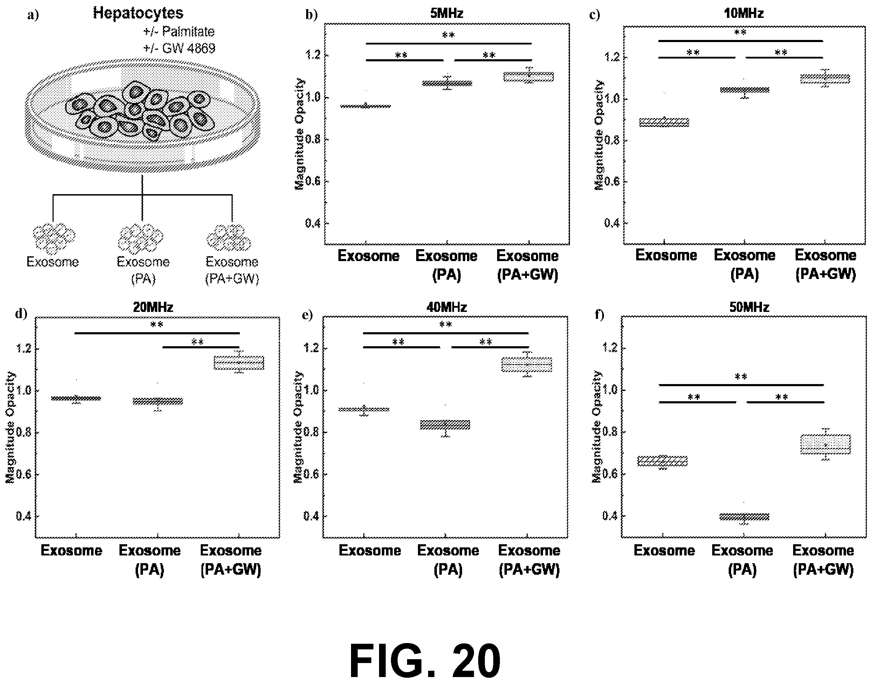

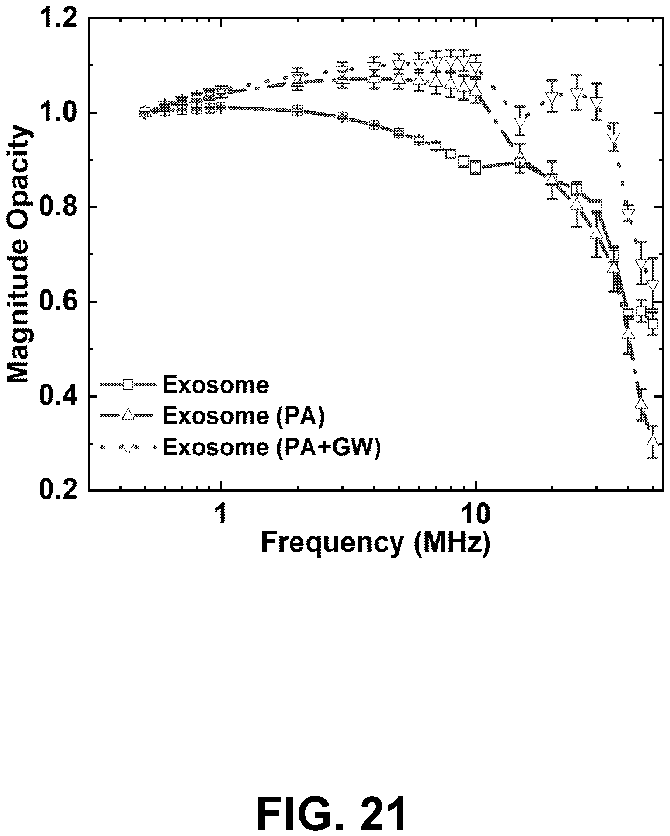

Human hepatocellular carcinoma (Huh 7) cells were cultured under three different conditions: 1) control, 2) PA (palmitate acid), and 3) PA+GW. Exosomes isolated from these three cell culture conditions were analyzed and were differentiated by the system. Figure 20A is an illustration showing the isolation of exosomes from hepatocytes under different culture conditions. Figures 20B-F are boxplots of the magnitude opacity for control exosomes, PA-treated exosomes and PA+GW-treated exosomes under the applied AC field at b) 5MHz; c) 10MHz; d) 20MHz; e) 30MHz; f) 50MHz. **$p<0.05.$* Figure 21 shows the opacity magnitude of the samples at a wide range of frequency.

Example 8

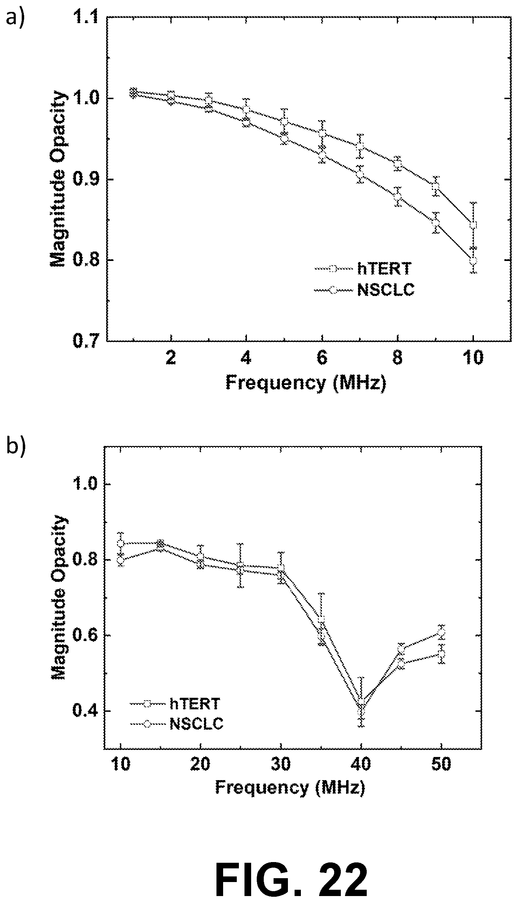

An opacity magnitude comparison was conducted of exosomes from hTERT mesenchymal stem cell exosomes and non-small cell lung cancer (NSCLC). Two types of exosomes were purchased from ATCC Inc: 1) hTERT mesenchymal stem cell exosomes, and 2) non-small cell lung cancer (NSCLC) exosomes. The results of the opacity magnitude comparison are shown in Figures 22a and 22b. FIG. 22a shows results at frequencies from 0-10 MHz. FIG. 22b shows results at frequencies from 10-50 MHz.

Example 9

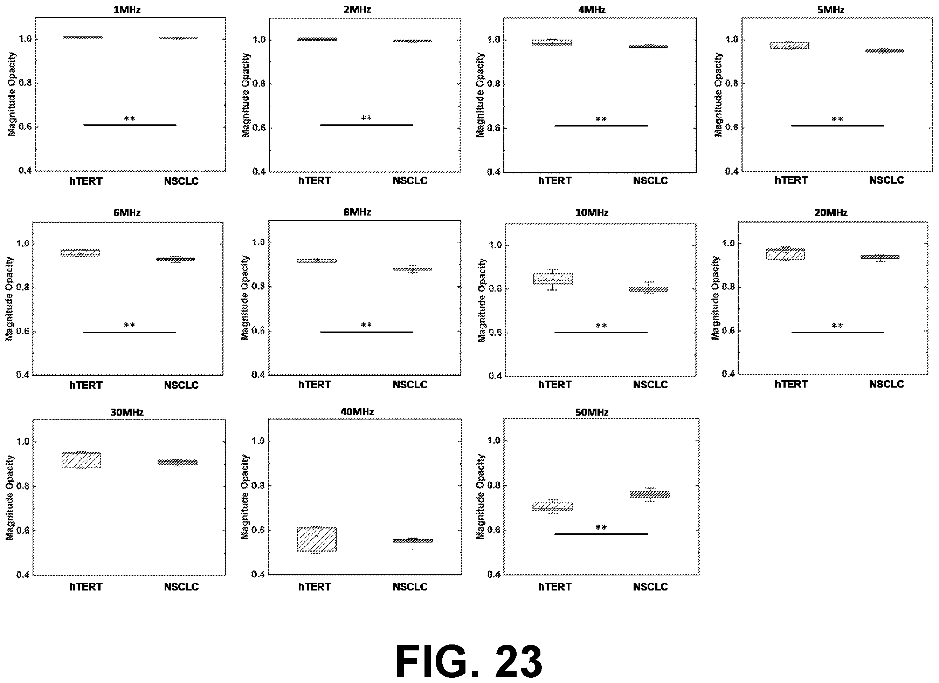

An opacity magnitude comparison was conducted of exosomes from hTERT mesenchymal stem cell exosomes and non-small cell lung cancer (NSCLC) under the applied AC field at 1MHz, 2MHz, 4MHz, 5MHz, 6MHz, 8MHz, 10MHz; 20MHz; 30MHz; 40MHz 50MHz. **$p<0.05.$* See Figure 23.

Example 10

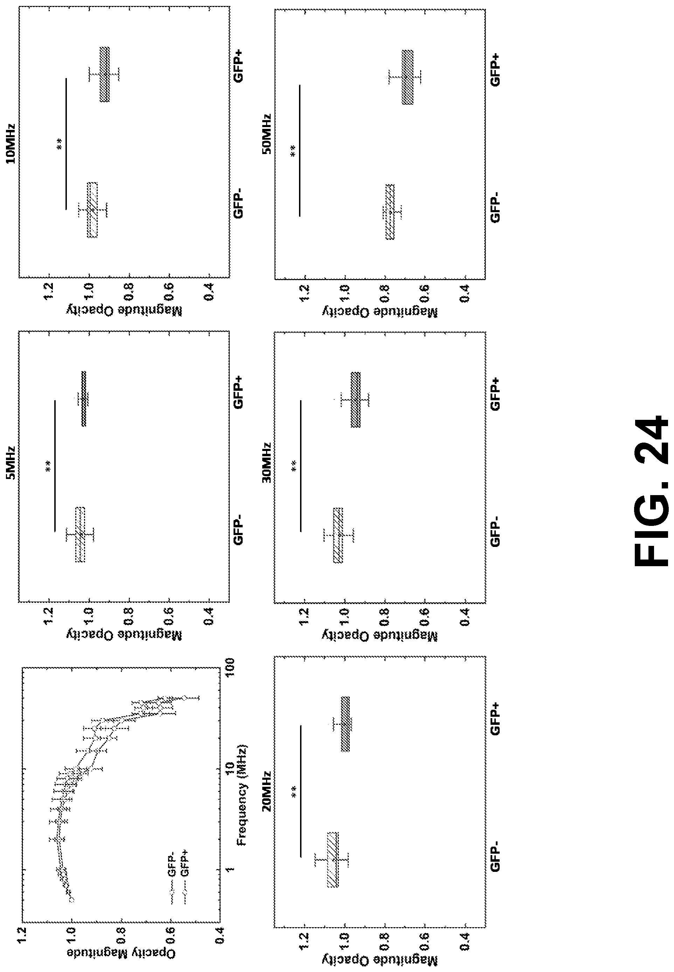

An opacity magnitude comparison was conducted for exosomes from culture media of mouse primary hepatocytes: 1) Wild-type (GFP-) sample, and 2) Green fluorescent protein-transgenic (GFP+) sample. Referring to Figure 24, a) shows an opacity magnitude comparison of exosomes derived of mouse primary hepatocytes under culture media without (GFP-exosome) and with green fluorescent protein-transgenic (GFP+ exosome). Figures 24b-f show boxplots of magnitude opacity for GFP- exosome and GFP+ exosome under the applied AC field at b) 5MHz; c) 10MHz; d) 20MHz; e) 30MHz; f) 50MHz. **$p<0.05.$* Significant difference was observed at frequency higher than 10MHz.

Example 11

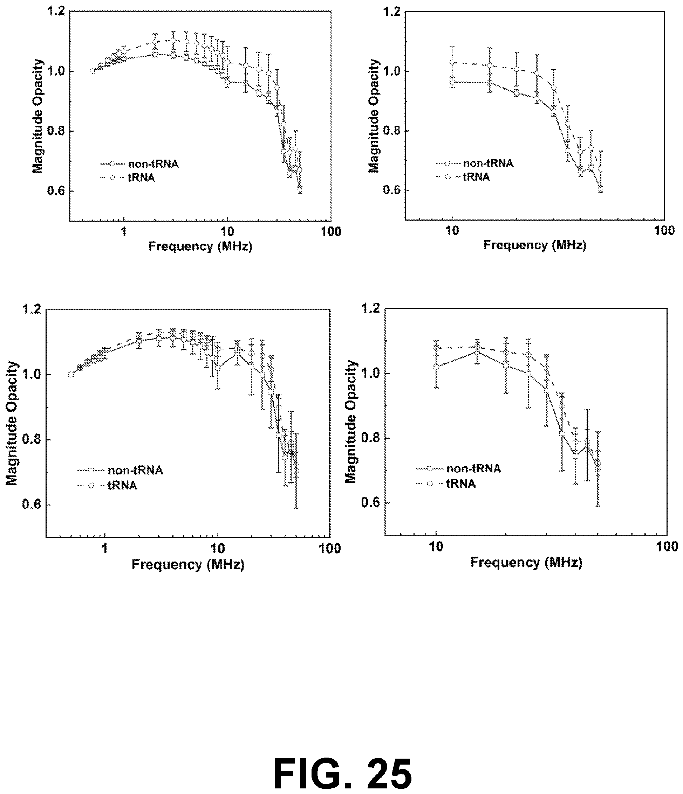

An opacity magnitude comparison was conducted of liposomes without and with transfer RNA encapsulate inside a) the concentration of liposomes detected is 5*10^8#/$\mu$L, and b) 5*10^6#/$\mu$L (see Figure 25). Total detection volume is 10 $\mu$L. The estimated number of tRNA molecules encapsulated per liposome is 426 oligonucleotide molecules. At a higher concentration of liposomes, the magnitude opacity showed significant difference between non-tRNA liposomes and tRNA encapsulated liposomes.

Example 12

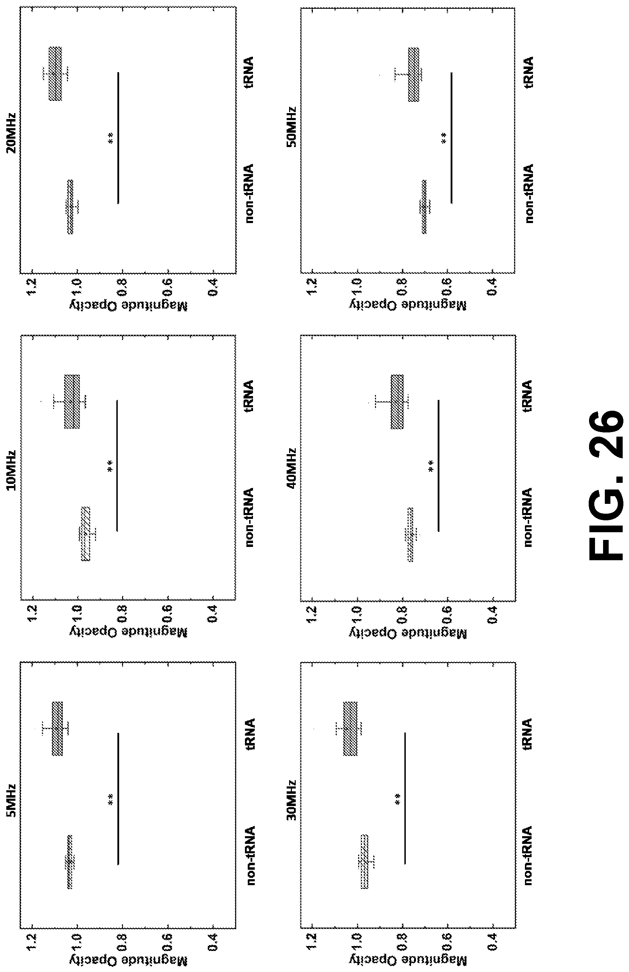

Figure 26 shows boxplots of magnitude opacity for liposomes without/with transfer RNA encapsulate inside under the applied AC field at a) 5MHz b)10MHz; c) 20MHz; d) 30MHz; e) 40MHz; f) 50MHz. **$p<0.05$.* The concentration of liposomes detected is $5*10^8\#/\mu L$.

Example 13

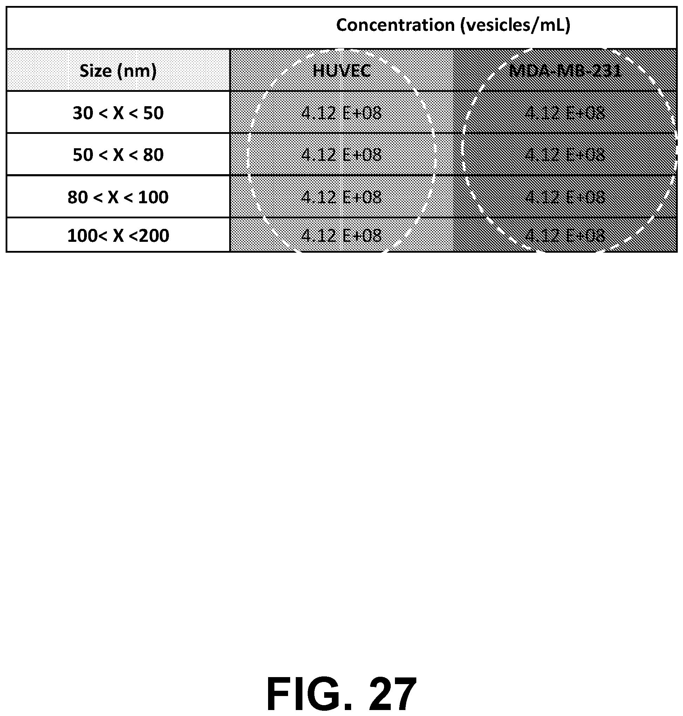

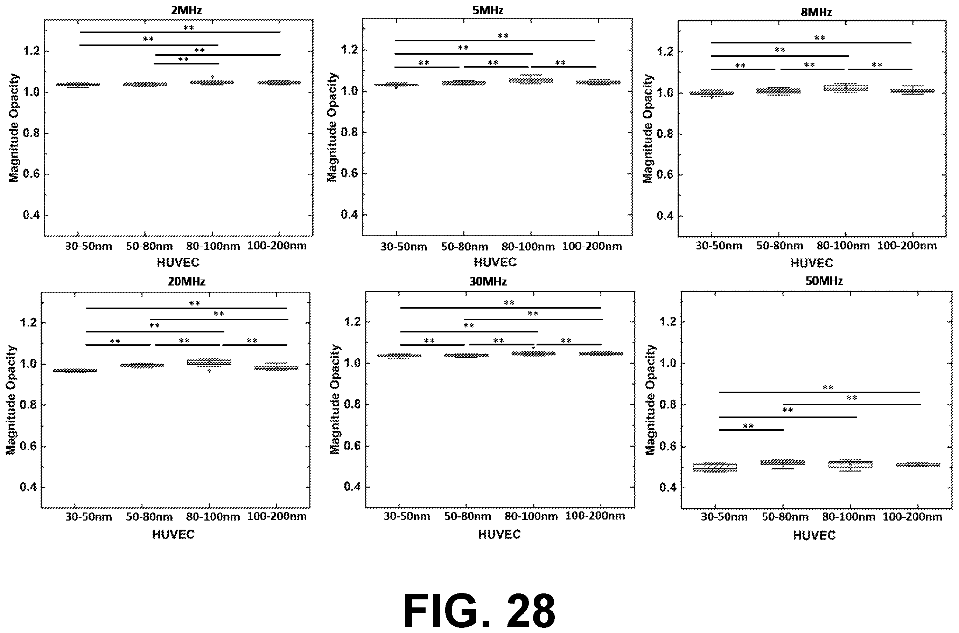

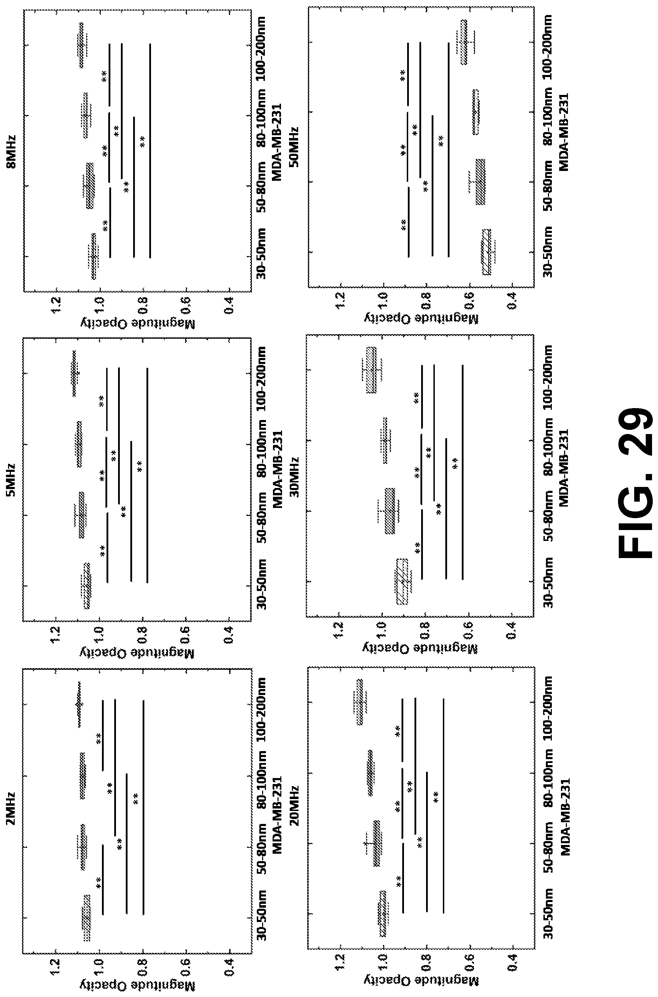

Tests were conducted to determine if exosomes at four size ranges could be discriminated. The exosomes were extracted from 1) HUVEC: Human Umbilical Vein Endothelial Cells, and 2) MDA-MB-231: breast cancer cells. Figure 27 shows the concentration of exosomes collected at four different size ranges for HUVEC and MDA-MB-231. An opacity magnitude comparison was conducted of the exosomes from HUVEC at different size ranges. Figure 28 shows boxplots of the magnitude opacity under the applied AC field at a) 2MHz b) 5MHz; c) 8MHz; d) 20MHz; e) 30MHz; f) 50MHz. **$p<0.05$. An opacity magnitude comparison was conducted of the exosomes from MDA-MB-231 at different size ranges. Figure 29 shows boxplots of the magnitude opacity under the applied AC field at a) 2MHz b) 5MHz; c) 8MHz; d) 20MHz; e) 30MHz; f) 50MHz. **$p<0.05$.

These experiments have shown distinct differentiation between exosomes and liposomes of similar size (100nm) by measuring their electrical impedance from 500 KHz to 50 MHz. Also, as a model system 100 nm liposomes were synthesized with different membrane compositions and cytosolic conductance to establish the cut off frequencies at which their membrane capacitance and inner lumen conductance are distinguishable. Our results show distinguishable impedance measurements of exosomes extracted from Human hepatocellular carcinoma (HuH-7) cells that were cultured at different culture medium conditions

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

It is to be further understood that where descriptions of various embodiments use the term "comprising," and / or "including" those skilled in the art would understand that in some specific instances, an embodiment can be alternatively described using language "consisting essentially of" or "consisting of."

It is therefore intended to cover in the appended claims all changes and modifications that are within the scope of this invention.

**Claims**

1. A method of characterizing nanovesicles comprising entrapping and sensing the dielectric properties of said nanovesicles **characterized in that** said method uses an electrical impedance sensing device (52) as defined in claim 13.

2. The method of claim 1 wherein the nanovesicles are selected from the group consisting of small extracellular vesicles, exosomes, liposomes, viruses and mixtures thereof.

3. The method of claim 1 wherein the nanovesicles comprise exosomes.

4. The method of claim 2 wherein the nanovesicles are liposomes and they are **characterized by** distinguishing between liposomes with different membrane compositions.

5. The method of claim 2 wherein nanovesicles are liposomes and they are **characterized by** distinguishing between liposomes loaded with RNA and liposomes without RNA.

6. The method of claim 3 wherein the exosomes are **characterized by** distinguishing between exosomes with different cytosolic compositions.

7. The method of claim 1 wherein the AC field is altered in magnitude and the results of the magnitude changes are analyzed to identify one or more biophysical dielectric properties of said exosomes.

8. The method of claim 1 wherein the AC field is altered in phase and the results of the phase changes are analyzed to identify one or more dielectric properties of said exosomes.

9. The method of claim 1 wherein the AC field is altered in magnitude and phase and the results of the magnitude and phase changes are analyzed to identify one or more dielectric properties of said exosomes.

10. The method of claim 1wherein the electrical impedance sensing device comprises an impedance analyzer, a power supply, a micromanipulator and a signal processor.

11. The method of claim 3 wherein the dielectric properties of said exosomes comprise opacity magnitude.

12. The method of claim 8 wherein the dielectric properties of said exosomes comprise opacity magnitude.

13. A device (52) for manipulating and analyzing particles (40), in particular nanovesicles, in a suspending medium, the device comprising:

a first chamber (60) configured to receive a back-fill medium;
a second chamber (58) configured to receive the suspending medium;
a nanopipette (54) including a first end (56) located in the first chamber and a second end (10) located in the second chamber, the first end including an inlet and the second end including a tip;
a first trapping electrode (28) located in the first chamber;
a second trapping electrode (30) located in the second chamber;
a first sensing electrode (47) located adjacent to the tip;
a second sensing electrode (48) located adjacent to the tip and opposing the first sensing electrode (47);
a signal source (36) including a first terminal electrically coupled to the first trapping electrode and a second terminal electrically coupled to the second trapping electrode, the signal source configured to output a reference signal (35) on the first terminal and a bias signal (34) on the second terminal, the reference signal and the bias signal defining an electrical signal having a characteristic that generates a potential well that traps the particles proximate to the tip of the nanopipette; and
an impedance amplifier electrically coupled to the first sensing electrode (47) and the second sensing electrode (48)
**characterized in that** the first and second sensing electrodes generate an AC field in the range of from about 500 KHz to about 50 MHz that interacts with the particles proximate to the tip of the nanopipette, producing an AC field signal, which is transmitted to a signal processor.

14. The device of claim 13 further comprising a micromanipulator configured to control the distance between the first sensing electrode and the second sensing electrode.

15. The method of claim 1 or the device of claim 13, wherein the AC field is in the range of from 1 MHz to 50 MHz.


**Patentansprüche**

1. Verfahren zum Charakterisieren von Nanovesikeln, umfassend Einschließen und Erfassen der dielektrischen Eigenschaften der Nanovesikel, **dadurch gekennzeichnet, dass** das Verfahren eine elektrische Impedanzerfassungsvorrichtung (52), wie in Anspruch 13 definiert, verwendet.

2. Verfahren nach Anspruch 1, wobei die Nanovesikel ausgewählt sind aus der Gruppe bestehend aus kleinen extrazellulären Vesikeln, Exosomen, Liposomen, Viren und Mischungen davon.

3. Verfahren nach Anspruch 1, wobei die Nanovesikel Exosomen umfassen.

4. Verfahren nach Anspruch 2, wobei die Nanovesikel Liposomen sind und sie **dadurch gekennzeichnet sind, dass** zwischen Liposomen mit unterschiedlichen Membranzusammensetzungen unterschieden wird.

5. Verfahren nach Anspruch 2, wobei Nanovesikel Liposomen sind und sie **dadurch gekennzeichnet sind, dass** zwischen mit RNA beladenen Liposomen und Liposomen ohne RNA unterschieden wird.

**6.** Verfahren nach Anspruch 3, wobei die Exosomen **dadurch gekennzeichnet sind, dass** zwischen Exosomen mit unterschiedlichen zytosolischen Zusammensetzungen unterschieden wird.

**7.** Verfahren nach Anspruch 1, wobei das AC-Feld in der Stärke verändert wird und die Ergebnisse der Stärkenänderungen analysiert werden, um eine oder mehrere biophysikalische dielektrische Eigenschaften der Exosomen zu identifizieren.

**8.** Verfahren nach Anspruch 1, wobei das AC-Feld in der Phase verändert wird und die Ergebnisse der Phasenänderungen analysiert werden, um eine oder mehrere dielektrische Eigenschaften der Exosomen zu identifizieren.

**9.** Verfahren nach Anspruch 1, wobei das AC-Feld in der Stärke und der Phase verändert wird und die Ergebnisse der Stärke- und Phasenänderungen analysiert werden, um eine oder mehrere dielektrische Eigenschaften der Exosomen zu identifizieren.

**10.** Verfahren nach Anspruch 1, wobei die elektrische Impedanzerfassungsvorrichtung einen Impedanzanalysator, eine Stromversorgung, einen Mikromanipulator und einen Signalprozessor umfasst.

**11.** Verfahren nach Anspruch 3, wobei die dielektrischen Eigenschaften der Exosomen eine Opazitätsstärke umfassen.

**12.** Verfahren nach Anspruch 8, wobei die dielektrischen Eigenschaften der Exosomen eine Opazitätsstärke umfassen.

**13.** Vorrichtung (52) zum Manipulieren und Analysieren von Partikeln (40), insbesondere Nanovesikeln, in einem Suspensionsmedium, wobei die Vorrichtung umfasst:

eine erste Kammer (60), welche dazu eingerichtet ist, ein Rückfüllmedium zu erhalten;
eine zweite Kammer (58), welche dazu eingerichtet ist, das Suspensionsmedium zu erhalten;
eine Nanopipette (54), umfassend ein erstes Ende (56), welches sich in der ersten Kammer befindet, und ein zweites Ende (10), welches sich in der zweiten Kammer befindet, wobei das erste Ende einen Einlass umfasst und das zweite Ende eine Spitze umfasst;
eine erste Einfangelektrode (28), welche sich in der ersten Kammer befindet;
eine zweite Einfangelektrode (30), welche sich in der zweiten Kammer befindet;
eine erste Erfassungselektrode (47), welche sich benachbart zu der Spitze befindet;
eine zweite Erfassungselektrode (48), welche sich benachbart zu der Spitze und gegenüber der ersten Erfassungselektrode (47) befindet;
eine Signalquelle (36), umfassend einen ersten Anschluss, welcher elektrisch an die erste Einfangelektrode gekoppelt ist, und einen zweiten Anschluss, welcher elektrisch an die zweite Einfangelektrode gekoppelt ist, wobei die Signalquelle dazu eingerichtet ist, ein Referenzsignal (35) auf dem ersten Anschluss und ein Vorspannungssignal (34) auf dem zweiten Anschluss auszugeben, wobei das Referenzsignal und das Vorspannungssignal ein elektrisches Signal definieren, welches eine Eigenschaft aufweist, welche eine Potentialmulde erzeugt, welche die Partikel in der Nähe der Spitze der Nanopipette einfängt; und
einen Impedanzverstärker, welcher elektrisch an die erste Erfassungselektrode (47) und die zweite Erfassungselektrode (48) gekoppelt ist,
**dadurch gekennzeichnet, dass** die erste und die zweite Erfassungselektrode ein AC-Feld im Bereich von etwa 500 kHz bis etwa 50 MHz erzeugen, welches mit den Partikeln in der Nähe der Spitze der Nanopipette interagiert und ein AC-Feldsignal geniert, welches an einen Signalprozessor übertragen worden ist.

**14.** Vorrichtung nach Anspruch 13, ferner umfassend einen Mikromanipulator, welcher dazu eingerichtet ist, den Abstand zwischen der ersten Erfassungselektrode und der zweiten Erfassungselektrode zu steuern.

**15.** Verfahren nach Anspruch 1 oder Vorrichtung nach Anspruch 13, wobei das AC-Feld im Bereich von 1 MHz bis 50 MHz liegt.

**Revendications**

**1.** Procédé de caractérisation de nanovésicules consistant à piéger et à détecter les propriétés diélectriques desdites nanovésicules, **caractérisé en ce que** ledit procédé utilise un dispositif de détection d'impédance électrique (52) tel que défini dans la revendication 13.

**2.** Procédé selon la revendication 1 dans lequel les nanovésicules sont sélectionnées parmi le groupe constitué de petites vésicules extracellulaires, d'exosomes, de liposomes, de virus et de mélanges de ceux-ci.

**3.** Procédé selon la revendication 1 dans lequel les nanovésicules comprennent des exosomes.

**4.** Procédé selon la revendication 2 dans lequel les nanovésicules sont des liposomes, **caractérisés par** la distinction entre des liposomes présentant des compositions de membrane différentes.

**5.** Procédé selon la revendication 2 dans lequel les nanovésicules sont des liposomes **caractérisés par** la distinction entre des liposomes chargés en ARN et des liposomes sans ARN.

**6.** Procédé selon la revendication 3 dans lequel les exosomes sont **caractérisés par** la distinction entre des exosomes sur la base de compositions cytosoliques différentes.

**7.** Procédé selon la revendication 1 dans lequel le champ alternatif est modifié en amplitude et les résultats des variations d'amplitude sont analysés pour identifier une ou plusieurs propriétés diélectriques biophysiques desdits exosomes.

**8.** Procédé selon la revendication 1 dans lequel le champ alternatif est modifié en phase et les résultats des variations de phase sont analysés pour identifier une ou plusieurs propriétés diélectriques desdits exosomes.

**9.** Procédé selon la revendication 1 dans lequel le champ alternatif est modifié en amplitude et en phase et les résultats des variations d'amplitude et de phase sont analysés pour identifier une ou plusieurs propriétés diélectriques desdits exosomes.

**10.** Procédé selon la revendication 1 dans lequel le dispositif de détection d'impédance électrique comprend un analyseur d'impédance, une alimentation électrique, un micromanipulateur et un processeur de signal.

**11.** Procédé selon la revendication 3 dans lequel les propriétés diélectriques desdits exosomes comprennent une amplitude d'opacité.

**12.** Procédé selon la revendication 8 dans lequel les propriétés diélectriques desdits exosomes comprennent une amplitude d'opacité.

**13.** Dispositif (52) destiné à manipuler et à analyser des particules (40), en particulier des nanovésicules, dans un milieu de suspension, le dispositif comprenant :

une première chambre (60) configurée pour recevoir un milieu de remblai ;
une seconde chambre (58) conçue pour recevoir le milieu de suspension ;
une nanopipette (54) incluant une première extrémité (56) située dans la première chambre et une seconde extrémité (10) située dans la seconde chambre, la première extrémité incluant une entrée et la seconde extrémité incluant un embout ;
une première électrode de piégeage (28) située dans la première chambre ;
une seconde électrode de piégeage (30) située dans la seconde chambre ;
une première électrode de détection (47) située adjacente à l'embout ;
une seconde électrode de détection (48) située adjacente à l'embout et opposée à la première électrode de détection (47) ;
une source de signal (36) incluant une première borne électriquement couplée à la première électrode de piégeage et une seconde borne électriquement couplée à la seconde électrode de piégeage, la source de signal étant conçue pour émettre un signal de référence (35) sur la première borne et un signal de polarisation (34) sur la seconde borne, le signal de référence et le signal de polarisation définissant un signal électrique présentant une caractéristique qui génère un puits de potentiel qui piège les particules à proximité de l'embout de la nanopipette ; et
un amplificateur d'impédance électriquement couplé à la première électrode de détection (47) et à la seconde électrode de détection (48)
**caractérisé en ce que** les première et seconde électrodes de détection génèrent un champ alternatif dans la plage d'environ 500 KHz à environ 50 MHz qui interagit avec les particules à proximité de l'embout de la nanopipette, produisant ainsi un signal de champ alternatif, qui est transmis à un processeur de signal.

14. Dispositif selon la revendication 13 comprenant en outre un micromanipulateur conçu pour commander la distance entre la première électrode de détection et la seconde électrode de détection.

15. Procédé selon la revendication 1 ou dispositif selon la revendication 13, dans lequel le champ alternatif est dans la plage de 1 MHz à 50 MHz.

FIG. 1

FIG. 2

FIG. 3

Nanopipette

EOF

DEP

EP

Low DC voltage

V<0

Exosome

# FIG. 4

FIG. 5

FIG. 6

EP 3 959 512 B1

**FIG. 7A**

# FIG. 7B

FIG. 8

FIG. 9

** p < 0.05

**FIG. 10**

# FIG. 11

i)

ii)

## FIG. 12A

## FIG. 12B

:lecithin (PC)    :cholesterol (CH)

**1:10** ratio of
cholesterol/lecithin

**10:1** ratio of
cholesterol/lecithin

# FIG. 13

**FIG. 14**

**FIG. 15**

**FIG. 16**

# FIG. 17

**FIG. 18**

**FIG. 19**

FIG. 20

FIG. 21

a)

b)

FIG. 22

**FIG. 23**

FIG. 24

EP 3 959 512 B1

# FIG. 25

FIG. 26

EP 3 959 512 B1

| Size (nm) | Concentration (vesicles/mL) | |
|---|---|---|
| | HUVEC | MDA-MB-231 |
| 30 < X < 50 | 4.12 E+08 | 4.12 E+08 |
| 50 < X < 80 | 4.12 E+08 | 4.12 E+08 |
| 80 < X < 100 | 4.12 E+08 | 4.12 E+08 |
| 100< X <200 | 4.12 E+08 | 4.12 E+08 |

# FIG. 27

**FIG. 28**

**FIG. 29**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62838015

- US 2008105565 A